# EUROPÄISCHE PATENTSCHRIFT

(11) **EP 0 641 228 B1**
(45) Veröffentlichungstag und Bekanntmachung des Hinweises auf die Patenterteilung: **13.08.1997**
(21) Anmeldenummer: 93909650.9
(22) Anmeldetag: 19.05.1993
(51) Int. Cl.: A61M 5/50

(54) **INJEKTIONSEINRICHTUNG FÜR EINMALVERWENDUNG**
SINGLE USE INJECTION DEVICE
DISPOSITIF D'INJECTION A USAGE UNIQUE

(30) Priorität: 19.05.1992 AT 1029/92
(43) Veröffentlichungstag der Anmeldung: 08.03.1995
(73) Patentinhaber: Pharmaplan GmbH, 61352 Bad Homburg v.d.H. (DE)
(72) Erfinder: MEYER, Philippe, CH-8021 Zürich (CH); PICKHARD, Ewald, A-1160 Wien (AT)
(74) Vertreter: Fuchs, Luderschmidt & Partner Patentanwälte
(86) Internationale Anmeldenummer: AT9300085
(87) Internationale Veröffentlichungsnummer: WO9323099

(56) Entgegenhaltungen:
- WO-A-92/04064
- FR-A- 2 632 190

## Beschreibung

Die Erfindung betrifft eine Injektionseinrichtung, wie sie im Oberbegriff des Patentanspruches 1 beschrieben ist.

Bei einer bekannten Injektionseinrichtung - gemäß FR-A- 26 32 190 - ist zur Verhinderung der Mehrfachverwendung der Injektionseinrichtung eine Originalitätssicherungsvorrichtung vorgesehen. Diese Originalitätssicherungsvorrichtung ist zwischen dem den Kolben aufnehmenden Innenraum der Injektionseinrichtung und der Injektionsnadel untergebracht und weist einen Käfig und ein Rückhalteglied für ein Umschaltorgan auf, das in Richtung des den Kolben aufnehmenden Innenraums des Spritzenzylinders mit einer Durchströmöffnung versehen ist, dessen Durchmesser kleiner ist als ein Durchmesser des als Kugel ausgebildeten Umschaltorganes. Das auf der der Injektionsnadel zugewandten Stirnseite des Käfigs angeordnete Rückhalteglied ist, im vorliegenden Fall eine elastisch verformbare Scheibe mit einer Bohrung deren Innendurchmesser kleiner als der Außendurchmesser der Kugel ist. Wird nun nach dem Aufziehen des Injektionsmediums in den Innenraum des Spritzenzylinders dieses Injektionsmedium durch die Injektionsnadel ausgedrückt, so wirkt auf das an der elastisch verformbaren Scheibe anliegende Umschaltorgan der volle Spritzendruck, wodurch aufgrund der großen Oberfläche der das Sperrorgan bildenden Kugel diese unter elastischer Aufweitung der Öffnung der Scheibe durch diese hindurchgepreßt wird und in eine daran anschließende Zwischenkammer fällt. Nach dem Durchtritt der Kugel durch die elastische Scheibe kann nun das Injektionsmedium durch die Öffnung in der elastischen Scheibe und an der Kugel vorbei in die Kanüle bzw. die Längsbohrung der Injektionsnadel eintreten und somit injiziert werden. Soll nun mit der Injektionseinrichtung ein weiteres Injektionsmedium aufgezogen werden, so wird mit dem Kolben im Innenraum des Spritzenzylinders ein Unterdruck erzeugt, der aufgrund der großen Fläche der Kugel bewirkt, daß das Sperrorgan nunmehr von der Injektionsnadel her an die elastisch verformbare Scheibe herangeführt wird und an dieser anliegt und somit einen dichtenden Abschluß bildet. Damit ist das weitere Aufziehen eines Injektionsmediums in den Innenraum des Spritzenzylinders verhindert. Sowohl im Bereich des Käfigs als auch im Bereich der Zwischenkammer, die die Kugel nach dem Durchtritt durch die elastische Scheibe aufnimmt, können in Richtung der Längsachse der Injektionseinrichtung verlaufende Stege bzw. Fortsätze und Vorsprünge vorgesehen sein, um ein einwandfreies Vorbeiströmen des Injektionsmediums in den verschiedenen Lagen des Sperrorgans beim Aufziehen des Mediums in den Innenraum vor der Benutzung der Injektionseinrichtung und beim Auspressen des Injektionsmediums durch die Injektionsnadel während dem Injizieren sicherzustellen. Bei entsprechend langer Gestaltung jenes Teils des Zwischenraums, der sich zwischen dem Käfig und der Injektionsnadel befindet, kann in Abhängig vom Bewegungsraum der Kugel in Richtung der Längsachse der Injektionseinrichtung ein Aspirieren ermöglicht werden. Nachteilig ist bei dieser Ausbildung, daß eine sichere Funktion der Injektionseinrichtung durch Verwendung einer elastischen Scheibe nicht gewährleistet ist und für jede Größe einer Injektionseinrichtung unterschiedliche Einbauteile benötigt werden.

Eine gleichartige weitere bekannte Injektionseinrichtung - gemäß der WO-A- 92/04064 - weist ebenfalls zur Verhinderung der Mehrfachverwendung der Injektionseinrichtung eine Originalitätssicherungsvorrichtung auf. Die Originalitätssicherungsvorrichtung ist wiederum zwischen den den Kolben aufnehmenden Innenraum der Injektionseinrichtung und der Injektionsnadel untergebracht und weist einen aus elastisch verformbarem Material, z.B. einem Elastomer bestehenden Käfig für das durch eine Kugel gebildete Rückhalteglied auf. In dem Käfig ist ein durch einen umlaufenden Dichtbund gebildetes Rückhalteglied ausgeformt, wobei ein Durchmesser der von dem Rückhalteglied umschlossenen Bohrung kleiner ist, als der Außendurchmesser der Kugel. Dieser aus dem elastisch verformbaren Material, z.B. dem Elastomer gebildete Käfig mit dem damit auch elastisch verformbaren Rückhalteglied ist durch dieses Rückhalteglied in eine erste und zweite Aufnahmekammer für die Kugel, die aus harten, relativ unflexiblem Material, wie z.B. Glas oder rostfreiem Stahl besteht, ist in einer aus zwei Gehäuseteilen bestehendes Gehäuse eingesetzt, welches in dem der Injektionsnadel zugewandten Endbereich des Innenraums des Spritzenzylinders angeordnet ist. Gleich wie bei der zuvor beschriebenen FR-OS 26 32 190 wird eine Wiederverwendung der Injektionseinrichtung nach einem einmaligen Injektionsvorgang dadurch verhindert, daß beim Auspressen des Injektionsmediums aus dem Innenraum des Spritzenzylinders durch den auf die Kugel ausgeübten Flüssigkeitsdruck diese durch elastische Verformung des Käfigs bzw. des Rückhaltegliedes des als Ventileinsatzstück ausgebildeten elastisch verformbaren Käfigs die Kugel durch dieses Rückhalteglied von der ersten Kammer in die zweite Kammer durchgedrückt wird. Diese Verformung des Rückhaltegliedes ist durch eine sich von der ersten in die zweite Kammer konisch verjüngende Ausbildung des Rückhaltegliedes begünstigt. Soll nun ein weiteres Injektionsmedium durch die zweite Kammer in die erste Kammer und damit in den Innenraum des Spritzenzylinders aufgesaugt werden, wird durch den beim Zurückziehen des Kolbens im Innenraum des Spritzenzylinders entstehenden Unterdruck die Kugel nunmehr von der nadelseitigen zweiten Kammer her gegen das durch den umlaufenden Bund gebildete Rückhalteglied gedrückt, das in einer ungefähr senkrecht zur Längsachse der Spritze verlaufenden Ebene angeordnet ist. Der beim Aufziehen des Kolbens erzeugte Unterdruck reicht dann nicht aus, um das Rückhalteglied gegen den elastischen Widerstand zu verformen und einen Durchtritt der Kugel von der zweiten Kammer in die erste Kammer zu erlauben. Vielmehr wird durch die Kugel die Öffnung des Rückhaltegliedes verschlossen und das weitere Aufziehen eines Injektionsmediums in den Innenraum des Spritzenzylinders ist damit verhindert. Desweiteren sind Ausführungsbeispiele beschrieben, bei welchen der Käfig aus einem harten unelastischem Material besteht und das Umschaltorgan aus einem in radialer Richtung elastischen Werkstoff besteht, der auch in Längsrichtung des Umschaltorganes mehrere hintereinander angeordnete Dichtabschnitte aufweisen kann. Nachteilig ist bei Injektionseinrichtungen gemäß den beiden vorgenannten Druckschriften, daß nach dem Auspressen einer bereits geringen Menge an Injektionsmedium aus dem Innenraum des Spritzenzylinders, die gegebenenfalls nur geringfügig größer ist als die zum Aspirieren benötigte Menge an Injektionsmedium die Originalitätssicherungsvorrichtung zuverlässig ausgelöst wird und andererseits nach dem Auslösen der Injektionseinrichtung eine sichere Abdichtung zwischen der Injektionsnadel und dem Innenraum des Spritzenzylinders - der ein weiteres Aufziehen eines Injektionsmediums zur mehrmaligen Verwendung der Injektionseinrichtung verhindern soll - sichergestellt wird. Darüber hinaus soll die Handhabung beim Injizieren von Injektionsmedien in einen Körper gleich sein, wie bei den bisher bekannten Injektionseinrichtungen.

Weiters sind auch bereits verschiedene Injektionseinrichtungen mit den unterschiedlichsten Einbauteilen und Konstruktionen bekannt, die eine Zerstörung der Injektionseinrichtung nach der ersten Verwendung sicherstellen. Dadurch soll eine Mehrfachverwendung der Injektionseinrichtungen, insbesondere in Ländern mit geringem Hygienebewußtsein und damit die Ansteckung mit seuchenartigen Krankheiten, insbesondere Aids, vermieden werden. So sind derartige Injektionseinrichtungen unter anderem aus der WO-A 90/03816 und WO-A 90/03817 bekannt.

Der vorliegenden Erfindung liegt die Aufgabe zugrunde eine Injektionseinrichtung zu schaffen, bei der ein zwischen einer Ruhestellung und einer Sperrstellung verstellbares Umschaltorgan einen sicheren Durchtritt des Umschaltorgans bei geringen Auslösekräften und eine zuverlässige Abdichtung des Innenraums gegen die Zufuhr eines weiteren Injektionsmediums durch Aufziehen des Kolbens und den dabei erzeugten Unterdruck bzw. durch Druckbefüllung ermöglicht wird.

Diese Aufgabe der Erfindung wird durch die im Kennzeichenteil des Patentanspruches 1 angegebenen Merkmale gelöst. Der überraschende Vorteil dieser Lösung liegt vor allem darin, daß durch die Halterung des Rückhaltegliedes in einem steifen, stabilen und elastisch nicht verformbaren Gehäuse eine exakte Positionierung unter konzentrischer Ausrichtung der Öffnung im Rückhalteglied auf die Längsmittelachse der Injektionseinrichtung sichergestellt wird, sodaß eine gleichmäßig dichte Anlage der Stirnfläche der Öffnung an den entsprechenden Berührungsflä chen des Umschaltorganes bzw. dessen Dichtansatz gegeben ist. Durch diese konzentrische Ausrichtung der Öffnung des Rückhaltegliedes wird dieses gleichzeitig aber auch konzentrisch zu der im harten Gehäuse angeordneten Führungsbahn für den Führungsansatz des Umschaltorganes ausgerichtet. Gleichzeitig wird durch die Ausbildung eines Führungsansatzes, der annähernd spielfrei in der Führungsbahn des Gehäuses geführt ist, sichergestellt, daß auch das Umschaltorgan selbst bzw. dessen Dichtabschnitt konzentrisch zur Öffnung ausgerichtet ist und am gesamten Umfang ein gleichbleibender Anlagedruck zwischen Umschaltorgan und Rückhalteglied und somit eine einwandfreie Abdichtung zwischen diesen beiden Teilen sowohl vor dem Durchtritt des Umschaltorgans durch das Rückhalteglied als auch nach dem Durchtreten des Dichtabschnittes des Umschaltorganes durch das Rückhalteglied sichergestellt. Diese überraschende Erkenntnis der vorliegenden Erfindung bewirkt aber nunmehr, daß vor allem durch die exakte Abdichtung zwischen dem Rückhalteglied und dem Umschaltorgan bzw. dessen Dichtabschnitt vor dem Durchtritt derselben durch das Rückhalteglied eine immer gleichbleibende Vorschubkraft vom Kolben der Injektionseinrichtung über das Medium auf das Umschaltorgan übertragen werden kann und dadurch sichergestellt wird, daß das Umschaltorgan bei der ersten Betätigung bzw. am Beginn des Austreibens des Mediums aus dem Innenraum des Spritzenzylinders das Umschaltorgan bzw. dessen Dichtabschnitt zuverlässig das Rückhalteglied passiert und damit die Zerstörung der Originalitätssicherungsvorrichtung eingeleitet bzw. die Injektionseinrichtung für eine nochmalige Verwendung unbrauchbar gemacht wird. Ein weiterer überraschender Vorteil dieser scheinbar einfachen erfindungsgemäßen Lösung liegt darin, daß durch die Auslegung der Originalitätssicherungsvorrichtung sowohl der Führungsansatz als auch die Führungsbahn im Gehäuse aus einem harten Material mit entsprechend günstigen Gleiteigenschaften ausgebildet sein kann, wodurch der Reibungswiderstand, also jene Kraft, die einem ordnungsgemäßen Durchtreten des Umschaltorganes bzw. dessen Dichtabschnitt durch das Rückhalteglied entgegenwirkt, möglichst klein gehalten werden kann. Dadurch kann mit den bei normalen Injektionseinrichtungen üblichen Auspreßdrücken für das Medium auch eine einwandfreie Auslösung bzw. Wirkung der Originalitätssicherungsvorrichtung sichergestellt werden. Dabei kann gleichzeitig auf die Verwendung und den Einsatz von zusätzlichen Gleitmitteln zwischen dem Umschaltorgan und dem Gehäuse bzw. dem Rückhalteglied verzichtet werden, wodurch die Verwendung dieser Injektionseinrichtungen auch in Verbindung mit Medien möglich ist, deren Einsatz gemeinsam mit derartigen Gleitmitteln nicht möglich ist. Weiters wird in vorteilhafter Weise erreicht, daß durch die konzentrische Ausrichtung der Öffnung des Rückhaltegliedes auf die Längsachse der Führungsbahn nach dem Durchtritt des Dichtungsabschnittes des Umschaltorganes das Rückhalteglied bzw. dessen Stützring am Führungsansatz zentriert geführt werden kann und durch die Anlage desselben am Führungsansatz ein freies Ausströmen des Mediums aus dem Innenraum des Spritzenzylinders verhindert und unmittelbar bei jeder entgegen der Ausspritzrichtung des Mediums ausgeübten Bewegung des Kolbens ein Rückströmen des Mediums in den Innenraum des Spritzenzylinders sofort unterbunden wird. Diese ständige Unterstützung und zentrische Ausrichtung des Stützrings des Rückhaltegliedes bewirkt aber andererseits, daß auch nach einer längeren Nichtbenutzung einer bereits gebrauchten Injektionseinrichtung der Stützring so exakt positioniert ist, daß bei einem versuchten Aufziehen eines Mediums zur nochmaligen Verwendung der Injektionseinrichtung der Dichtabschnitt auch dann einen bleibenden dichten Abschluß durch die dichte Anlage am Rückhalteglied bewirkt, wodurch eine hohe Betriebssicherheit der Originalitätssicherungsvorrichtung erreicht wird. Dadurch wird auch verhindert, daß die Injektionseinrichtung beispielsweise durch Druckbefüllung wieder befüllt werden kann. Dies wird dadurch begünstigt, daß der Entleerdruck höher ist, als der Ansaugunterdruck. Darüberhinaus wird bei Einsatz der erfindungsgemäßen Injektionseinrichtung auch das Totvolumen unter der man jene Menge an Medium versteht, die nach dem vollständigen Auspressen des Mediums aus dem Innenraum des Spritzenzylinders - also dann wenn der Kolben an der vorderen Stirnseite der Originalitätssicherungsvorrichtung anliegt - zwischen der der Injektionsvorrichtung zugewandten Stirnseite des Kolbens und der Austrittsöffnung der Injektionsnadel verbleibt und nicht mehr aus der Injektionseinrichtung ausgepreßt werden kann. Die Auslegung der Originalitätssicherungsvorrichtung ist so getroffen, daß auch bei Verwendung derselben die von den Normen vorgeschriebenen Maximalwerte für dieses Totvolumen nicht überschritten werden.

Vorteilhaft ist auch eine Ausgestaltung nach Patentanspruch 2, wodurch eine kippsichere Führung des Umschaltorgans erzielt wird. Dadurch wird die einseitige Belastung auf das Rückhalteglied verringert und eine sichere klemmfreie Auslösung des Umschaltorgans erreicht.

Eine weitere mögliche Ausbildung beschreibt Patentanspruch 3, wodurch auch eine Herstellung des Umschaltorgans zur Führung im Gehäuse mit etwas gröberen Toleranzen möglich ist.

Die Weiterbildung nach Patentanspruch 4 zeichnet sich dadurch aus, daß vor allem Verklemmungen vor dem Durchtritt des Dichtabschnittes durch das Rückhalteglied zwischen dem Umschaltorgan und dem Rückhalteglied vermieden werden.

Vorteilhaft ist auch eine weitere Ausgestaltung nach Patentanspruch 5, da damit einerseits ein einwandfreies Entlüften der erfindungsgemäßen Injektionseinrichtung erzielt wird und nicht gleichzeitig eine Fehlauslösung des Umschaltorgans erfolgt bevor nicht ein entsprechender Druck zum Austreiben des Mediums mit der Injektionseinrichtung erzeugt ist, und andererseits die Originalitätssicherungsvorrichtung nicht aktiviert werden kann.

Die Ausgestaltung nach Patentanspruch 6 gestattet neben der Beaufschlagung des Umschaltorgans auf der dem Innenraum zugewandten Stirnfläche auch ein Anströmen mit dem über den Kolben unter Druck gesetzten Medium in radialer Richtung erfolgt, wodurch die zur Druckbeaufschlagung vorhandene Fläche vergrößert und damit ein sicheres Auslösen der Originalitätssicherungsvorrichtung erreicht wird.

Durch die Ausführungsvariante nach Patentanspruch 7 wird die Herstellung der Originalitätssicherungsvorrichtung mit einer geringen Anzahl von Einzelteilen und relativ einfach in den Spritzenzylinder einbaubar ermöglicht.

Eine vorteilhafte Weiterbildung wird durch die Ausgestaltung nach Patentanspruch 8 erreicht, wodurch eine automatische Montage ermöglicht wird.

Möglich ist aber auch eine Ausbildung nach Patentanspruch 9, wodurch eine eine automatische Montage erleichternde Baugruppenbildung erreicht wird.

Eine weitere vorteilhafte Ausgestaltung beschreibt Patentanspruch 10, wodurch die Gehäuseteile kostengünstig verbunden werden können.

Eine vorteilhafte Weiterbildung, wie sie Patentanspruch 11 beschreibt, ermöglicht das Ausbilden sehr wirkungsvoller Dichtflächen bei geringer Länge für den Dichtabschnitt.

Möglich ist auch eine Ausbildung nach Patentanspruch 12, wodurch ein entsprechender Aufweiteeffekt für das Hindurchtreten des Dichtorgans durch den Stützring bereits bei geringen Flüssigkeitsströmen erreicht wird.

Es sind aber auch vorzugsweise Ausführungen nach Patentanspruch 13 und 14 möglich, womit eine strömungsgünstige Abstufung der Querschnitte in Flußrichtung erreicht wird.

Eine vorteilhafte weitere Ausgestaltung beschreibt Patentanspruch 15, wodurch im montierten Zustand das Umschaltorgan auf eine größere Distanz, als es der Länge der Führungsbahn entspricht, zentrisch geführt ist und nach dem Umschalten ein verläßlicher Sitz für das Abdichten zur Verhinderung einer neuerlichen Befüllung erreicht wird.

Möglich ist dabei auch eine Ausbildung nach Patentanspruch 16, weil dadurch der Umschaltvorgang erleichtert wird.

Nach der Ausbildung entsprechend Patentanspruch 17 wird ein unbehindertes, elastisches Verformen des Stützringes beim Umschaltvorgang erreicht.

Nach einer vorteilhaften Weiterbildung entsprechend Patentanspruch 18 oder 19 wird eine einwandfreie Wirkungsweise auch beim Auftreten fertigungstechnisch bedingter Maßabweichungen für die Elemente erreicht.

Entsprechend der Ausbildung nach Patentanspruch 20 wird eine unbeabsichtigte Rückstellung in die Ausgangsstellung des Umschaltorgans wirkungsvoll verhindert, wobei der Widerstand gegen eine Rückstellung in etwa linear mit einer Druckbelastung, die für einen derartigen Vorgang aufgebracht wird, ansteigt.

Eine vorteilhafte Weiterbildung beschreibt Patentanspruch 21, wodurch zusätzliche Bauelemente und damit Kosten eingespart werden.

Durch die Ausbildung nach Patentanspruch 22 wird eine lagesichere Halterung des Umschaltorgans in Sperrstellung erreicht.

Möglich ist dabei eine Ausbildung nach Patentanspruch 23, wodurch die einwandfreie Wirkungsweise der einer Wiederbefüllung entgegenstehenden Bauteilen keine zusätzliche Betätigungskraft erfordert bzw. eine Erhöhung dieser nicht eintritt.

Vorteilhaft ist aber auch eine Ausbildung nach Patentanspruch 24, wodurch eine gute Anpassung in der Kraftwirkung für die Rückstellung bei standardmäßig unterschiedlich großen Injektionseinrichtungen erreicht wird.

Von Vorteil ist aber auch eine Ausbildung nach Patentanspruch 25, womit eine Positionierung im Innenraum im Bereich der der Injektionsnadel zugewandten Stirnwand und in Längsrichtung der Injektionseinrichtung erreicht wird.

Durch die Ausbildung, wie sie im Patentanspruch 26 beschrieben wird, ist der Aspirationsvorgang durch eine Längsverschieblichkeit des Gehäuses mit dem Umschaltorgan möglich, ohne daß aufwendige, konstruktive und bauliche Maßnahmen erforderlich sind.

Nach der Ausbildung entsprechend Patentanspruch 27 kann das für einen einwandfreien Aspirationsvorgang erforderliche Volumen auf die erforderlichen Gegebenheiten abgestimmt werden.

Die vorteilhafte Weitergestaltung entsprechend Patentanspruch 28 ermöglicht ein Axialspiel des Umschaltorgans in seiner Sperrstellung, das den Aspirationsvorgang ermöglicht, jedoch darüber hinaus eine Wiederbefüllung der Injektionseinrichtung wirkungsvoll verhindert wird.

Nach einer vorteilhaften Weiterbildung entsprechend Patentanspruch 29 wird das vollständige Auspressen von eventuell mitangesaugter Luft vor der Verabreichung des Injektionsmediums erreicht.

Entsprechend einer Weiterbildung, wie sie Patentanspruch 30 beschreibt, wird bereits im Fertigungsvorgang eine einwandfreie Konzentrizität der Führungsbahn zum Innenraum des Spritzenzylinders erreicht, die von Fertigungstoleranzen weiterer Bauteile, wie z.B. des Gehäuses für das Umschaltorgan nicht beeinflußt wird.

Nach der vorteilhaften Ausbildung entsprechend Patentanspruch 31 wird die Wirkungsweise der erfindungsgemäßen Injektionseinrichtung verläßlich erreicht.

Vorteilhaft ist jedoch auch eine Ausbildung, wie sie Patentanspruch 32 beschreibt, weil eine derartige Ausbildung vorteilhaft für die Ausgestaltung der für die Fertigung erforderlichen Werkzeuge ist.

Möglich ist aber auch eine Ausbildung nach Patentanspruch 33, weil dadurch auch bei großvolumigeren Injektionseinrichtungen flache Ansatzwinkel bei der Verabreichung des Injektionsmediums erreicht werden.

Entsprechend einer vorteilhaften Weiterbildung nach Patentanspruch 34 wird eine Abstimmung der Durchflußmöglichkeiten zwischen einem Medium niederer Viskosität, wie z.B. Luft und einem Medium höherer Viskosität, also dem Applikationsmedium erreicht.

Nach einer Weiterbildung entsprechend Patentanspruch 35 wird eine kostengünstige Herstellungsmöglichkeit für den Durchtrittskanal erreicht.

Schließlich ist aber auch eine Ausbildung nach Patentanspruch 36 vorteilhaft, weil dadurch der Durchtrittsquerschnitt für den Durchtrittskanal auf eine größere Umfangslänge des Rückhaltegliedes verteilt wird, wodurch ungleichmäßige Strömungsverhältnisse vermieden werden.

Die Erfindung wird im nachfolgenden anhand der in den Zeichnungen dargestellten Ausführungsbeispiele näher beschrieben.

Es zeigen:
- Fig. 1: eine erfindungsgemäß ausgebildete Injektionseinrichtung in Seitenasicht und vereinfachter, schematischer Darstellung;
- Fig. 2: einen Teil der Injektionseinrichtung nach Fig.1 in Seitenansicht geschnitten und vergrößertem Maßstab, in einer Stellung des Umschaltorgans zum Aufziehen eines Mediums in den Innenraum des Spritzenzylinders;
- Fig. 3: die Injektionseinrichtung im Bereich der Führungsbahn in Stirnansicht geschnitten, gemäß den Linien III-III in Fig.2;
- Fig. 4: die Injektionseinrichtung im Bereich des Rückhaltegliedes in Stirnansicht geschnitten gemäß den Linien IV-IV in Fig.2;
- Fig. 5: die Injektionseinrichtung nach den Fig.2 bis 4 während des Auspressens des Mediums durch die Injektionsnadel:
- Fig. 6: die Injektionseinrichtung nach den Fig.2 bis 5 bei dem Versuch nach dem Auspressen eines Mediums erneut ein Medium für eine weitere Injektion in den Innenraum des Spritzenzylinders aufzuziehen;
- Fig. 7: eine andere Ausführungsform einer erfindungsgemäßen Injektionseinrichtung mit einem an den Dichtabschnitt angeformten Dichtansatz in Seitenansicht geschnitten;
- Fig. 8: eine andere Ausführungsvariante des Umschaltorgans mit einem über den Dichtungsabschnitt in Richtung der Injektionsnadel fortspringenden Dichtungsansatz in Seitenansicht geschnitten;
- Fig. 9: die Injektionseinrichtung nach Fig.8 im Bereich der parallel zu, Führungsbahn verlaufenden Kanäle in Stirnansicht geschnitten, gemäß den Linien IX-IX in Fig. 8;
- Fig.10: das Gehäuse mit der Führungsbahn für das Umschaltorgan bei weggelassenem Rückhalteglied in Draufsicht;
- Fig.11: das Gehäuse in Seitenansicht geschnitten, gemäß den Linien XI-XI in Fig.10;
- Fig.12: einen Teil einer anderen Ausführungsform der Injektionseinrichtung in Seitenansicht geschnitten und vergrößertem Maßstab, in einer Stellung des Umschaltorganes zum Aufziehen eines Mediums in den Innenraum des Spritzenzylinders;
- Fig.13: die Injektionseinrichtung nach Fig.12 in der Stellung des Umschaltorgans zum Entlüften des Innenraums des Spritzenzylinders;
- Fig.14: die Injektionseinrichtung nach Fig.12 oder 13 im Bereich des Umschaltorgans mit der Stellung des Umschaltorgans und des Rückhaltegliedes beim Auspressens des Mediums;
- Fig.15: die Injektionseinrichtung nach Fig.12 bis 14 beim Versuch nach dem Auspressen des ursprünglich aufgezogenen Mediums erneut ein Medium in den Innenraum des Spritzenzylinders auf zuziehen;
- Fig.16: die Injektionseinrichtung nach den Fig.12 bis 15 beim Zurück ziehen des Kolbens zum Aspirieren;
- Fig.17: Injektionseinrichtung in Stirnansicht geschnitten gemäß den Linien XVII-XVII in Fig.16;
- Fig.18: die Injektionseinrichtung in Stirnansicht geschnitten, gemäß den Linien XVIII-XVIII in Fig.16;
- Fig.19: die Injektionseinrichtung in Stirnansicht, geschnitten, gemäß den Linien XIX-XIX in Fig.16;
- Fig.20: eine andere Ausbildung einer erfindungsgemäßen Injektionsein richtung mit einer anderen Ausführungsvariante eines Entlüftungskanals in Seitenansicht geschnitten, mit einem im vorderen Endbereich des Spritzenzylinders angeordneten Aufnahmeraum für das Umschaltorgan;
- Fig.21: die Injektionseinrichtung nach Fig. 20 in Stirnansicht geschnitten, gemäß den Linien XXI-XXI in Fig.20
- Fig.22: einen Teil einer anderen Ausführungsvariante einer erfindungsgemäßen Injektionseinrichtung im Bereich der Originalitätssicherungsvorrichtung in Seitenansicht geschnitten;
- Fig.23: einen Teil einer anderen Ausführungsform einer erfindungsgemäßen injektionsvorrichtung im Bereich des Umschaltorgans in Seitenansicht geschnitten.

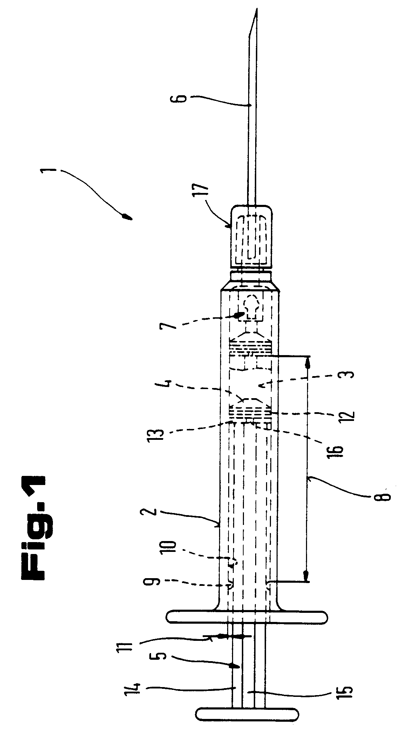

In Fig.1 ist eine Injektionseinrichtung 1 gezeigt, die einen Spritzenzylinder 2 und einen in einem Innenraum 3 desselben verfahrbaren Kolben 4, der mit einer Kolbenstange 5 betätigbar ist, aufweist. In einem einer Injektionsnadel 6 zugewandten Auslaßbereich des Spritzenzylinders 2 ist eine schematisch eingezeichnete Originalitätssicherungsvorrichtung 7 vorgesehen. Mit dieser Originalitätssicherungsvorrichtung 7 soll eine Mehrfachverwendung der Injektionseinrichtung 1 verhindert werden.

Darunter ist zu verstehen, daß nach einem Aufziehen eines ersten Mediums zum Injizieren in eine beispielsweise leer gelieferte Injektionseinrichtung 1 und dessen Auspressen im Zuge eines Injektionsvorgangs kein weiteres Injektionsmedium mehr in den Innenraum 3 der Injektionseinrichtung 1 aufgesaugt bzw. eingepreßt werden kann.

Durch diese Verhinderung der Mehrfachverwendung soll vor allem eine Infizierung mit ansteckenden Krankheiten, vor allem Aids bei Impfungen oder bei Abgabe von Dauermedikamenten, wie beispielsweise Insulin verhindert werden.

Diese Originalitätssicherungsvorrichtung 7, die im nachfolgenden anhand einer Injektionseinrichtung 1 beschrieben wird, bei der in bevorzugter Weise der Spritzenzylinder 2 aus Kunststoff besteht, kann natürlich auch bei Injektionseinrichtungen 1 mit vorzugsweise silikonisierten Spritzenzylindern 2 aus Glas eingesetzt werden.

Dabei ist es aber auch möglich, den Spritzenzylinder 2 mit einer Kupplungsvorrichtung für beliebig aufsteckbare Injektionsnadeln, beispielsweise mit einem Luer-Konus zu versehen um die Injektionsnadeln 6, die in einer Schutzhülle angeordnet sein können, erst unmittelbar vor Verwendung der Injektionseinrichtung 1 aufzustecken. Andererseits ist es selbstverständlich auch möglich, daß die Injektionsnadel 6 direkt mit dem Spritzenzylinder 2 verbunden, z.B. eingeklebt, eingespritzt oder eingegossen ist.

Ein Hub 8 des Kolbens 4 ist in der der Injektionsnadel 6 abgewandten Richtung durch Anschläge 9, die über eine Innenfläche 10 des Spritzenzylinders 2 vorragen, begrenzt. Eine Höhe 11 dieser Anschläge 9 ist derart bemessen, daß sie den Kolben 4 im Bereich eines umlaufenden Kolbenringes 12, insbesondere eines O-Ringes zurückhalten. Es ist aber ebenso möglich, daß der Anschlag 9 direkt mit einem Kolbenkörper 13 zusammenwirkt und dazu entsprechend weit zwischen die meist um 90° zueinander versetzten Stege 14,15 der Kolbenstange 5 eingreift.

Bei entsprechender Ausbildung der Höhe 11 der Anschläge 9 kann sichergestellt werden, daß bei dem Versuch, den Kolben 4 aus dem Innenraum 13 des Spritzenzylinders 2 herauszuziehen, die über die Kolbenstange 5 ausgeübte Zugkraft so hoch ist, daß eine Schwächungsstelle 16 zwischen Kolbenstange 5 und Kolben 4 diese Zugkraft auf den Kolben 4 nicht mehr übertragen kann und abbricht. Damit ist sichergestellt, daß eine mißbräuchliche Verwendung der Injektionseinrichtung 1 auch durch Herausziehen des Kolbens 4 und Befüllen eines Innenraums 3 von der Kolbenstangensei te her unterbunden ist.

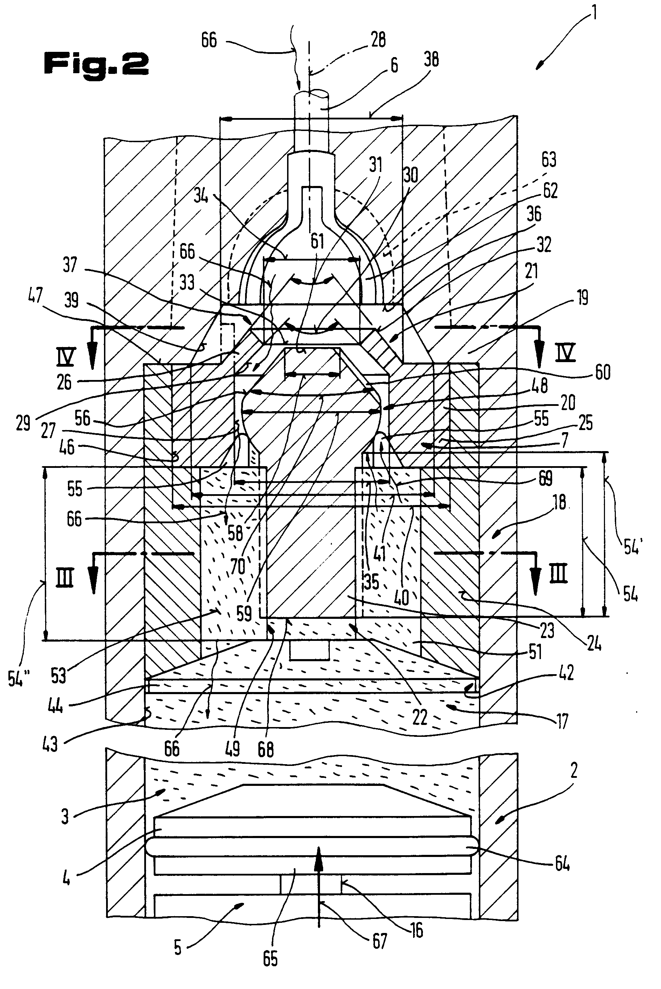

In Fig.2 ist nur ein Teil der Injektionseinrichtung 1 in größerem Maßstab dargestellt. So ist in Fig.2 nur der der Injektionsnadel 6 zugewandte Endbereich eines Innenraums 3 des Spritzenzylinders 2 dargestellt.

Die Originalitätssicherungsvorrichtung 7 umfaßt ein Gehäuse 18, zwischen welchem und einem Ansatz 19 im Spritzenzylinder 2 bevorzugt ein als Scheibe 20 ausgebildetes Rückhalteglied 21 für das Umschaltorgan 22 mit einem Führungselement 23 angeordnet ist. Die Originalitätssicherungsvorrichtung 7 kann auch einen einteiligen Bauteil bilden, wobei dann das Gehäuse 18 mit dem Rückhalteglied 21 einen einstückigen Bauteil bildet. Durch die Aufteilung der Originalitätssicherungsvorrichtung 7 in das Gehäuse und das Rückhalteglied 21 können jeweils die am besten geeigneten Materialien für den jeweiligen Anwendungsfall verwendet werden.

Üblicher Weise besteht das Gehäuse 18 aus einem härteren Kunststoff 24, auf der beispielsweise ein Elastizitätsmodul von über 2000 N/mm² aufweist bzw. eine Härte Shore A von größer 90 und eine Shore Härte D von größer 70 aufweist. Gleichermaßen besteht das Umschaltorgan 22 ebenfalls aus einem derart harten Kunststoff, insbesondere einem thermoplastischen oder duroplastischen Kunststoff, wobei sich sowohl für das Gehäuse 18, als auch für das Umschaltorgan 22 duroplastische Kunststoff empfehlen, da sie wenig temperaturabhängig und wenn sie ausgehärtet sind in organischen Lösungsmitteln unzersetzlich sind.

Dem gegenüber besteht das Rückhalteglied 21 bzw. die Scheibe 20, welche bei dieser Ausführung bevorzugt als einstückiger Bauteil ausgebildet sind, aus einem weicheren Kunststoff 25, nämlich einem sogenannten Elastomer. Dies ist ein Formstoff, der in einem ausreichenden Gebrauchs-Temperaturbereich und Raumtemperatur sich weich gummielastisch verhält. Geringe Spannungen bewirken beträchtliche Verformungen, nach Aufhebung der Spannung stellen diese sich bis nahezu auf die ursprünglichen Dimensionen zurück. Sie werden auch bei Temperaturerhöhung nicht fließbar, sondern sie verhalten sich elastomer bis zur Grenztemperatur irreversiblen chemischen Abbaus. Es können dafür chemisch vernetzte Elastomer-Erzeugnisse oder thermoplastische Elastomere verwendet werde. Die Härte Shore A liegt zwischen 20 und 60.

Selbstverständlich ist es auch möglich, daß nicht die gesamte Scheibe 20 aus diesem Kunststoff 25 bzw. dem Elastomer besteht, sondern lediglich ein Stützring 26, der über eine Durchgangsbohrung 27 der Scheibe in Richtung einer Längsachse 28 der Injektionseinrichtung 1 vorragt. In diesem Fall ist es vorteilhaft, wenn der Stützring 26 an der Scheibe 20 oder unter Wegfall der Scheibe am Gehäuse 18 oder einem Gehäuseteil angeformt ist.

Der Stützring ist als Hohlkegelabschnitt ausgebildet und weist eine Innenfläche 29, einen Öffnungswinkel 30 auf, der größer ist, als ein Öffnungswinkel 31 einer Außenfläche 32. Eine Öffnung 33 weist einen Durchmesser 34 auf, der kleiner ist, als ein Durchmesser 35 der Durchgangsbohrung 27. Der Stützring 26 ragt in einen Freiraum 36 der sich in einem Einlaßbereich 37 der Injektionseinrichtung 1 befindet und sich von den Ansätzen 19 in Richtung der Injektionsnadel 6 erstreckt. Weiters ist dieser Freiraum 36 so bemessen, bzw. weist eine Weite 38 auf, die es gestattet, daß der Stützring 26 auch dann, wenn sich seine Innenflächen 29 parallel zur Längsachse 28 erstrecken, eine Außenfläche 32 nicht an einer Begrenzungswand 39 des Freiraums anliegt, wie dies in strichpunktierten Linien in Fig.2 gezeigt ist.

Der Stützring 26, der als Hohlkegelabschnitt ausgebildet ist, weist bevorzugt im Bereich seiner Öffnung 33 eine größere Wanddicke auf, als an der Basis des Hohlkegelabschnittes. Dabei hat es sich als vorteilhaft herausgestellt, wenn die Wanddicke an der Basis in etwa 0,2 mm beträgt und sich auf in etwa 0,3 mm im Bereich der Öffnung 33 konisch erweitert. Damit wird einerseits ein günstiges Umformverhalten bei bevorzugt geringeren Stellkräften im Übergangsbereich zu der Scheibe 20 bzw. dem Gehäuse 18 erreicht und andererseits eine ausreichende Breite der Dichtfläche im Stirnendbereich für die Anlage und einen einwanfrei dichten Abschluß zwischen dem Rückhalteglied 21 und dem Umschaltorgan 22 erzielt. Die stabilere Dichtfläche aufgrund der größeren Breite verhindert auch eine Druckbefüllung über die Injektionsnadel 6.

Dagegen ist ein Außendurchmesser 40 der Scheibe 20 größer, als ein Innendurchmesser 41 des Freiraumes 36, sodaß die Scheibe 20 und das Gehäuse 18 sich gegen die Ansätze 19 im Spritzenzylinder 2 abstützen. Die Originalitätssicherungsvorrichtung 7 ist im Einlaßbereich 37 des Spritzenzylinders 2 dadurch unverschiebbar positioniert, da sich die Scheibe 20 und das Gehäuse 18 einerseits an den Ansätzen 19 an der Stirnwand des Spritzenzylinders 2 abstützen und in Gegenrichtung, also zum Kolben 4 zu, das Gehäuse über einen Anschlagteil 42, beispielsweise eine über eine Innenfläche 43 des Spritzenzylinders 2 vorspringenden Bund 44 anliegt. Das Gehäuse 18 ist somit zwischen den Ansätzen 19 und dem Anschlag 42 eingespannt. Das Einsetzen des Gehäuses 18 in den Einlaßbereich 37 des Spritzenzylinders 2 erfolgt durch Einpressen des Gehäuses 18, wobei entweder das Gehäuse 18 oder der Spritzenzylinder 2 entsprechend verformt wird, bis das Gehäuse 18 hinter dem Anschlagteil 42 eingerastet ist.

Die Scheibe 20 ist ihrerseits in einer Ausnehmung 46, in einer Stirnseite 47 des Gehäuses 18 eingesetzt, sodaß die den Ansätzen 19 zugewandte Stirnfläche der Scheibe 20 bevorzugt bündig bzw. ebenflächig mit der Stirnseite 47 verläuft.

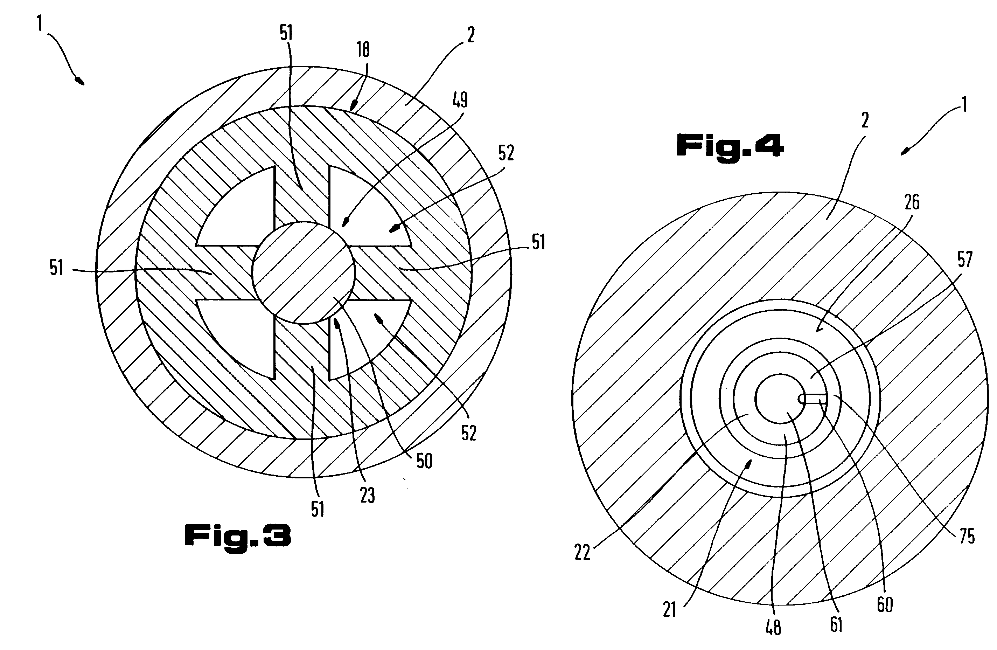

Das Umschaltorgan 22 besteht seinerseits aus einem Dichtabschnitt 48 und dem Führungselement 23. Dieses Führungselement 23 ist in einer Führungsbahn 49 im Gehäuse 18 geführt. Wie besser aus Fig.3 zu ersehen ist, wird diese Führungsbahn 49 für das durch einen Führungszapfen 50 gebildete Führungselement 23 durch unter 90° zueinander versetzte Führungsstege 51 des Gehäuses 18 gebildet. Zwischen den einzelnen Führungsstegen 51 sind Kanäle 52 ausgebildet, durch welche ein Medium 53 vom Innenraum 3 der Injektionseinrichtung 1 zur Injektionsnadel 6 hindurchtreten kann. Die Führungsbahn 49 wird somit durch eine teilweise unterbrochene durch die Führungsstege 51 gebildete Führungsbohrung hergestellt, in der der Führungszapfen 50 in Richtung der Längsachse 28 spielfrei verschiebbar ist. Diese Führungsbahn 49 ist bevorzugt mit einer sehr genauen Passung bzw. mit Toleranzen, die im Bereich kleiner 0,1 mm, bevorzugt im Bereich von 0,01 mm liegen, hergestellt, sodaß über eine gesamte Führungslänge 54, über welche sich eine Führungslänge 54 des Führungselementes 23 und eine Führungslänge 54 der Führungsbahn 49 überdecken, eine zentrische Führung des Umschaltorgans 22 gewährleistet ist.

Diese Genauigkeit der Führung zwischen dem Umschaltorgan 22 und der Führungsbahn 49 bewirkt, wie im folgenden noch näher erläutert werden wird, auch ein störungsfreies Zusammenspielen zwischen dem Stützring 26 und dem Dichtabschnitt 48 des Umschaltorganes 22.

Unterstützt wird diese zentrische Führung des Umschaltorgans 22 durch Führungsnasen 55, die von den Führungsstegen 51 in Richtung der Scheibe 20 vorragen und den Dichtabschnitt 48 im Bereich seiner beispielsweise durch einen Kugelkalottenabschnitt gebildeten Oberfläche 56 abstützen und zentriert haltern. Die Scheibe 20 kann mit entsprechenden Ausschnitten versehen sein, um die Führungsnasen aufzunehmen, es ist aber auch ebenso möglich, die Scheibe ohne Ausnehmungen herzustellen, wodurch die Führungsnasen 55 gleichzeitige Zentrierung der Scheibe 20 beim Einsetzen des Gehäuses 18 zwischen den Ansätzen 19 und dem Anschlagteil 42 eingesetzt wird. Damit wird auch eine Verlagerung der aus einem Elastomer bestehenden Scheibe 20, die elastisch verformbar ist, also somit einen biegeschlaffen Teil darstellt, auch bei einer längeren Lagerdauer bzw. eine verbesserte Einspannung der Scheibe vor allem beim Durchdrücken des Dichtabschnittes 48 durch die Öffnung 33 des Stützringes 26 erreicht.

Der nunmehr im Bereich der Oberfläche 56 als Kugelkalottenabschnitt auf der dem Führungselement 23 zugewendeten Seite ausgebildete Dichtabschnitt 48 ist in seinem dem Stützring 26 zugewandten Endbereich mit einer als Drehkegelstumpf ausgebildeten Oberfläche 57 versehen.

Ein Durchmesser 58 ist dabei kleiner als ein Innendurchmesser 34 der Öffnung 33, wogegen ein Außendurchmesser 59 des Dichtabschnittes 48 größer ist als der Durchmesser 34 der Öffnung 33. Vielfach entspricht der Außendurchmesser 59 dem Durchmesser des Kugelkalottenabschnittes der Oberfläche 56.

Die Funktionsweise der erfindungsgemäßen Injektionseinrichtung 1 wird im Folgenden anhand der Darstellungen in Fig. 1 bis 6 näher erläutert:

In der Oberfläche 57 des Drehkegelstumpfes 56 ist in Richtung einer Seitenkante bzw. parallel zu einer Seitenkante bzw. Oberfläche desselben verlaufend - wie insbesondere am Besten aus Fig.4 ersichtlich - ein Entlüftungskanal 60 angeordnet, der sich von der als Kugelkalottenabschnitt ausgebildeten Oberfläche 56 bis in den Bereich einer Stirnfläche 61 des Dichtabschnittes 48 erstreckt.

An den Freiraum 36 schließt in Richtung der Injektionsnadel 6 weiters eine Aufnahmekammer 62 zumindest für den Dichtabschnitt 48 des Umschaltorgans 22 an. Wie der zeichnerischen Darstellung, insbesondere in Fig.2 zu entnehmen ist, ist eine räumliche Ausbildung dieser Aufnahmekammer 62 derart gewählt, daß sie zumindest von ihren inneren Abmessungen den Außenabmessungen des Dichtabschnittes 48 entspricht. Zudem sind dieser Aufnahmekammer 62 benachbart Leitkanäle 63 angeordnet durch die das Injektionsmedium auch dann, wenn sich der Dichtabschnitt 48 in dieser Aufnahmekammer 62 befindet, an diesem außen in Richtung der Injektionsnadel 6 vorbei strömen kann.

Weiters ist beim dargestellten Ausführungsbeispiel ein Kolben 4 gezeigt, der mit einem als Kolbenring 12 ausgebildeten in den Kolben 4 beispielsweise aus Kunststoff eingesetzten O-Ring 64 gebildet ist. Es ist aus dieser Ausführung auch die Ausbildung des Schwächungsbereiches 16 zwischen der Kolbenstange 5 und dem Kolben 4 ersichtlich.

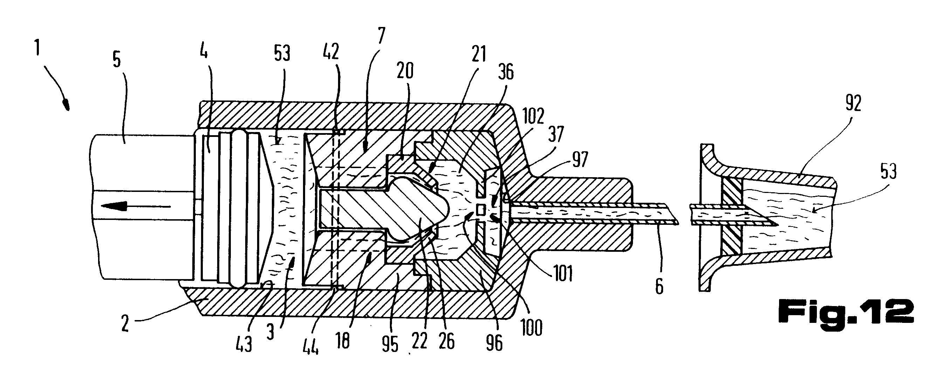

Wird nun wie beispielsweise besser aus Fig.2 ersichtlich aus einer nicht dargestellten Ampulle - wie beispielsweise für eine andere Ausführungsform in Fig.12 gezeigt ein Medium 53 in den Innenraum 3 des Spritzenzylinders 2 aufgesaugt, so ist dazu die Kolbenstange 5 mit dem daran befestigten Kolben 4 in Richtung des Pfeils 65 von der Injektionsnadel wegzubewegen. Dadurch wird im Innenraum 3 des Spritzenzylinders 2 ein Unterdruck aufgebaut der ein Zurückziehen des Umschaltorgans - auch dann falls das Umschaltorgan 22 während des Transportes der Injektionseinrichtung 1 an dem Stützring 26 angelegen hat, in die in Fig. 2 in vollen Linien gezeigte Stellung bewirkt. Gleichzeitig wird durch den, durch das Zurückziehen des Kolbens 4 bewirkten Unterdruck wie schematisch durch die Pfeile 66 angedeutet, wird das Medium 53 durch die Injektionsnadel 6 über die Aufnahmekammer 62 zwischen dem Stützring 26 und den Dichtabschnitt 48 des Umschaltorgans 22 hindurch, weiters durch die Kanäle 52 - Fig.3 - in den Innenraum 3 des Spritzenzylinders 2 aufgezogen.

Sobald die Ampulle geleert ist, bzw. eine ausreichende Menge am Medium 53 im Innenraum 3 vorhanden ist, kann dieses Medium nun dadurch aus dem Innenraum 3 ausgepreßt werden, daß der Kolben 4 mit der Kolbenstange 5 in Richtung des Pfeils 67 bewegt wird. Durch den sich dadurch aufbauenden Flüssigkeitsdruck wird über eine Stirnseite 68 des Umschaltorgans die dem Innenraum 3 des Spritzenzylinders 2 zugewandt ist, eine Vorschubkraft auf das Umschaltorgan 22 ausgeübt und entspricht dabei die ausgeübte Druckkraft, üblicherweise vorgesehenen Wert 5N - 10N, insbesondere 7,5N, so wird dadurch in jedem Fall sichergestellt, daß durch eine derart hohe Druckkraft, das Umschaltorgan 22 in Richtung der Injektionsnadel 6 vorwärts geschoben wird, bis dessen Oberfläche 57 an der Innenfläche 29 des Stützrings 26 des Rückhaltegliedes 21 dichtend anliegt.

Selbstverständlich genügt in der Praxis meist bereits ein wesentlich geringerer Wert der auszuübenden Druckkraft, der also erheblich unterhalb des Maximalwertes von 7,5N - 10N liegt, unmittelbar nach Betätigung des Kolbens 4 in Richtung des Pfeiles 67 einen dichten Abschluß zwischen dem Innenraum 3 und der Injektionsnadel 6 herzustellen, sodaß kein Medium 53 durch die Injektionsnadel 6 austreten kann. Diese kleinen Vorschubkräfte genügen üblicherweise deshalb, da das Umschaltorgan 22 neben der Stirnseite 68 auch noch gemäß dem Pfeil 69 über die Kanäle 52 der Dichtabschnitt 48, also der über dem Umfang des Führungselementes 23 vorspringende Teil im Bereich seiner Oberfläche 57 - wie schematisch durch Pfeile 69 angedeutet - tangential bzw. seitlich angeströmt wird und somit die durch das Medium 53 ausgeübte Summenkraft zur Erzeugung einer Vorschubbewegung des Umschaltorgans 22 in jedem Fall ausreicht, um eine Bewegung des Umschaltorgans in Richtung des Stützringes 26 sicherzustellen.

Bevor nun mit der erfindungsgemäßen Injektionseinrichtung 1 ein Medium 53 abgegeben werden kann, ist die Injektionseinrichtung 1 zu entlüften. Dazu wird üblicher Weise die Injektionsvorrichtung in eine schräge Position gebracht, in welcher sich die Injektionsnadel 6 am höchsten Punkt befindet. Daraufhin werden die im Injektionsmedium vorhandenen Lufteinschlüsse durch leichtes Klopfen auf den Spritzenzylinder 2 dazu gebracht, sich im oberen Endbereich zu sammeln bzw. aus der Injektionseinrichtung auszutreten. Dabei wird gleichzeitig durch den Benützer der Injektionseinrichtung 1 ein geringer Druck über die Kolbenstange 5 und den Kolben 4 auf das Medium 53 ausgeübt. Allerdings wird, bedingt durch die Ausgestaltung der erfindungsgemäßen Injektionseinrichtung 1, zwischen dem Umschaltorgan 22 und dem Stützring 26 ein exakter dichtender Abschluß bewirkt, der vor allem auch darauf zurückzuführen ist, daß der Öffnungswinkel 30 der Innenfläche 29 des Stützringes 26 größer ist, als ein Öffnungswinkel 70 des Drehkegelstumpfes des Umschaltorganes 22. Durch den geringeren Durchmesser 58 der Stirnfläche 61 wird beim Eintreten des Umschaltorganes 22 in die Öffnung 33 diese etwas aufgeweitet, bis sich die Innenfläche 29 mit dem geringeren Öffnungswinkel 30 satt an die Oberfläche 57 des als Drehkegelstumpf ausgebildeten Dichtabschnittes 48 anlegt.

Um nun trotzdem eine einwandfreie Entlüftung des Mediums 53 im Innenraum 3 des Spritzenzylinders 2 zu ermöglichen, ist entlang einer der Seitenkante des Drehkegelstumpfes zumindest ein Entlüftungskanal 60 vorgesehen. Der oder die Entlüftungskanäle erstrecken sich, wie in Fig.2 in strichlierten Linien angedeutet, dann von außerhalb des abgedichteten Bereiches bis in den Freiraum 36, sodaß die Luftblasen unter einem geringen Vorspanndruck mit dem Kolben 4 aus dem Innenraum 3 in Richtung der Injektionsnadel 6 und durch die durch Schläge auf den Spritzenzylinder 2 ausgeübten Vibrationen durch die Injektionsnadel 6 ins Freie austreten können.

Bei der Auslegung des bzw. der Entlüftungskanäle, es können nämlich auch mehreren Entlüftungskanäle 60 vorgesehen sein, ist auf die Viskosität des jeweiligen Injektionsmediums bedacht zu nehmen. Unter Ausnützung des Fachwissens ist nämlich der Querschnitt der jeweiligen Entlüftungskanäle 60 unter Miteinbeziehung der Viskosität der mit der Injektionseinrichtung 1 zu verspritzenden Injektionsmedien so abzustimmen, daß die Reibungsverluste aufgrund der Haftkraft der Flüssigkeit an der Oberfläche so bemessen werden, daß bei einer Ausübung einer Druckkraft durch den Benutzer auf die Kolbenstange 5 bzw. den Kolben 4, die unter jener Kraft liegt, die benötigt wird, um das Umschaltorgan 22 durch das Rückhalteglied 21 hindurchzubewegen, durch den Entlüftungskanal 60 kein Injektionsmedium hindurchtreten kann. Die Entlüftungskanäle bzw. deren Querschnittsform ist also so auszulegen, daß diese für die zu verwendenden flüssigen Injektionsmedien flüssigkeitsdicht sind.

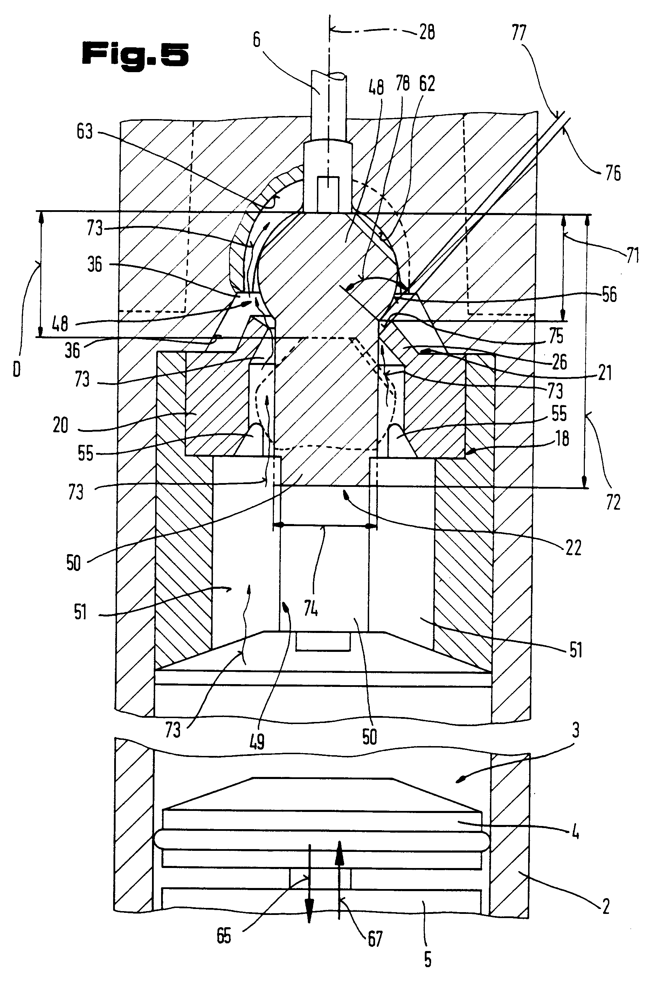

Gleichzeitig ist darauf Bedacht zu nehmen, daß der Querschnitt der Entlüftungskanäle beispielsweise bei sehr zähen Injektionsmedien nicht unter voller Ausnutzung der möglichen Querschnittsflächen gewählt wird, um einen zu starken Druckabbau zu vermeiden, der dazu führen würde, daß die mit dem Kolben 4 aufbringbare Druckkraft nicht ausreicht, um das Umschaltorgan 22 von der in Fig.2 gezeigten Ruhestellung in die in Fig.5 gezeigte Sperrstellung zu verstellen.

Wird dann der Druck des Benutzers auf den Kolben so stark erhöht, daß ein Injektionsmedium, beispielsweise in den Körper eines Patienten abgegeben werden kann, bzw. auch unabhängig davon durch die Injektionsnadel 6 ausgedrückt werden kann, so wird die durch die Pfeile 69 ausgeübte Vorschubkraft so stark erhöht, bis die Vorschubkraft ausreicht, um den Stützring 26, der als Dichtungsmembran wirkt, so weit elastisch in radialer Richtung und in Richtung der Injektionsnadel 6 aufzudehnen, daß der Dichtabschnitt 48 durch die Öffnung 33 im Stützring 26 hindurchtreten kann. Durch die gleichzeitige Führung des Umschaltorganes 22 mit dem Führungszapfen 50 in der Führungsbahn 49 wird eine gleichmäßige Aufweitung der Öffnung 33 über den gesamten Querschnittsbereich erzielt und vor allem verhindert, daß durch eine Verkantung des Dichtabschnittes 48 im Bereich seines kegelstumpfförmig ausgebildeten Endes das Injektionsmedium zwar durch die Öffnung 33 austreten kann, das Umschaltorgan 22 jedoch nicht durch die Öffnung 33 hindurchtritt.

Dazu ist die Führungslänge 54 so bemessen, daß sie größer ist, als eine Distanz D zwischen der in strichlierten Linien gezeigten Ruhestellung in Fig.5 und der in der gleichen Figur gezeigten vordersten Sperrstellung in Richtung der Injektionsnadel 6. Die in strichlierten Linien in Fig.5 gezeigte Stellung entspricht dabei der in vollen Linien gezeigten Stellung in Fig.2. Zumindest muß jedoch die Führungslänge 54 einer Länge 71 des Dichtabschnittes 48 entsprechen.

Dabei ist weiters noch zu berücksichtigen, daß die Führungslänge 54 so bemessen wird, daß über den gesamten Verstellweg bzw. die Distanz D eine kippsichere achsparallele Führung des Umschaltorgans 22 entlang bzw. parallel zur Längsachse 28 bzw. der Längsachse der Führungsbahn 49 sichergestellt ist.

Selbstverständlich ist es auch möglich, daß die Achse bzw. Längsachse der Führungsbahn 49 zwar parallel, jedoch seitlich versetzt gegenüber der Längsachse 28 der Injektionsnadel 6 oder der Injektionseinrichtung 1 verläuft. Dadurch kann verhindert werden, daß durch Einführen eines mechanischen Schuborgans oder dgl. von Seiten der Injektionsnadel 6 her das Umschaltorgan 22 aus seiner Sperrstellung mit Gewalt wieder in die Ruhestellung gemäß der Darstellung in Fig.2 zurückgedrückt werden kann. Es ist selbstverständlich aber auch möglich, daß durch den Einbau von gegeneinander versetzten und einander überdeckenden Umlenkplatten oder durch versetzte Schlitze ein mechanisches Zurückstellen des Umstellorgans 22 verhindert werden kann.

Des weiteren ist darauf zu achten, daß eine Gesamtlänge 72 des Umschaltorganes zumindest größer ist, als der Außendurchmesser 59 bzw. der maximale Außendurchmesser des Umschaltorgans 22. Nur dadurch kann gewährleistet werden, daß das Umschaltorgan 22 auch automatisch und selbsttätig bei der Montage zugeführt und montiert werden kann. Bevorzugt ist jedoch eine Führungslänge 54 des Führungszapfens 50 doppelt so lang als die Länge 71 des Dichtabschnittes 48. Dadurch wird auch bei der vordersten Extremstellung des Umschaltorganes 22 noch eine Führung desselben zwischen den Führungsstegen 51, also somit in der Führungsbahn 49 im Gehäuse 18 erreicht.

Nachdem das Umschaltorgan 22 durch den Stützring 26 hindurch in den Freiraum 36 und danach in die Aufnahmekammer 62 eingetreten ist, kann das Injektionsmedium, wie durch Pfeile 73 schematisch angedeutet, durch die Kanäle 52 an den Führungsnasen 55 vorbei, bis zum Stützring 26 durchtreten. Wird nun eine geringe oder überhaupt keine Vorschubkraft im Sinne des Pfeiles 67 aufgebracht, so liegt der Stützring 26 nachdem der Durchmesser 34 der Öffnung 33 kleiner ist, als ein Außendurchmesser 74 des Führungszapfens 50an diesem an und ist somit ein dichtender Abschluß zwischen den Führungszapfen 50 und dem Stützring 26 bzw. der durch diesen gebildeten Dichtungsmembran erreicht. Wird dagegen die Kraft in Richtung des Pfeiles 67 verstärkt, so drückt das Medium 53 im Sinne der Pfeile 73 von Innen her auf die Innenfläche 29 des Stützringes 26, wodurch die Dichtungsmembran aufgeweitet bzw. gedehnt wird und das Medium 53 am Dichtabschnitt 48 entlang durch die Leitkanäle 63 in die Injektionsnadel 6 durchtreten kann.

Ist dann das Injektionsmedium aus dem Innenraum 3 des Spritzenzylinders 2 ausgepreßt, und wird versucht, ein neues Injektionsmedium aufzuziehenunabhängig davon, ob der Innenraum 3 nun bereits völlig entleert ist oder nicht - so wird aufgrund des mit dem Kolben 4 dadurch ausgeübten Unterdrucks in Verbindung mit der elastischen Vorspannung der durch den Stützring 26 gebildeten Dichtungsmembran ein sofortiger dichtender Abschluß zwischen dem Stützring 26 bzw. dessen Stirnfläche 75 erreicht, sodaß auch bei einer Druckbefüllung, bei welcher also ein weiteres Injektionsmedium durch die Injektionsnadel 6 in den Innenraum 3 des Spritzenzylinders 2 eingepreßt werden soll, ein dichtender Abschluß erreicht wird

Wird der Unterdruck bzw. der über die Injektionsnadel 6 eingeführte Druck eines weiteren Injektionsmediums noch höher, so wird das Umschaltorgan 22 in Richtung des Pfeiles 65 zurückgezogen und zwar so weit, bis die Oberfläche 56 des als Kugelkalottenabschnitt ausgebildeten Teils des Umschaltorgans an der Stirnfläche 75 des Stützringes 26 anliegt. Dazu ist diese Stirnfläche 75 sich in Richtung der Injektionsnadel 6 konisch erweiternd ausgebildet, wobei eine Seitenkante 76 des Konuses parallel zu einer Geraden 77 verläuft, die sich unter einem Winkel 78 von 90° zu einer auf den Berührungsbereich zwischen der Stirnfläche 75 und dem Kugelkalottenabschnitt der Oberfläche 56 verlaufenden Bereich erstreckt. Damit bildet die Stirnfläche 75 eine Tangentenfläche zum Kugelkalottenabschnitt und somit ist dadurch ein exakt dichtender Abschluß zwischen dem als Dichtungsmembran ausgebildeten Stützring 26 und dem Dichtabschnitt 48 erreicht.

Bedingt durch diese Ausgestaltung der Stirnfläche 75 wird nun auch bei einer weiteren Erhöhung des Druckes, insbesondere auch durch die Ausführung der Oberfläche 56 als kugelkalottenförmige Oberfläche oder beispielsweise sich als in Richtung der Injektionsnadel erweitender Kegelstumpf oder dgl. ähnliche räumliche Ausbildung keine Spreizkraft in radialer Richtung auf den Stützring 26 aufgebracht, wodurch ein Aufweiten der Dichtungsmembran bzw. des Stützringes 26 für eine Bewegung des Umschaltorgans 22 in Richtung des Pfeils 65 zuverlässig verhindert ist.

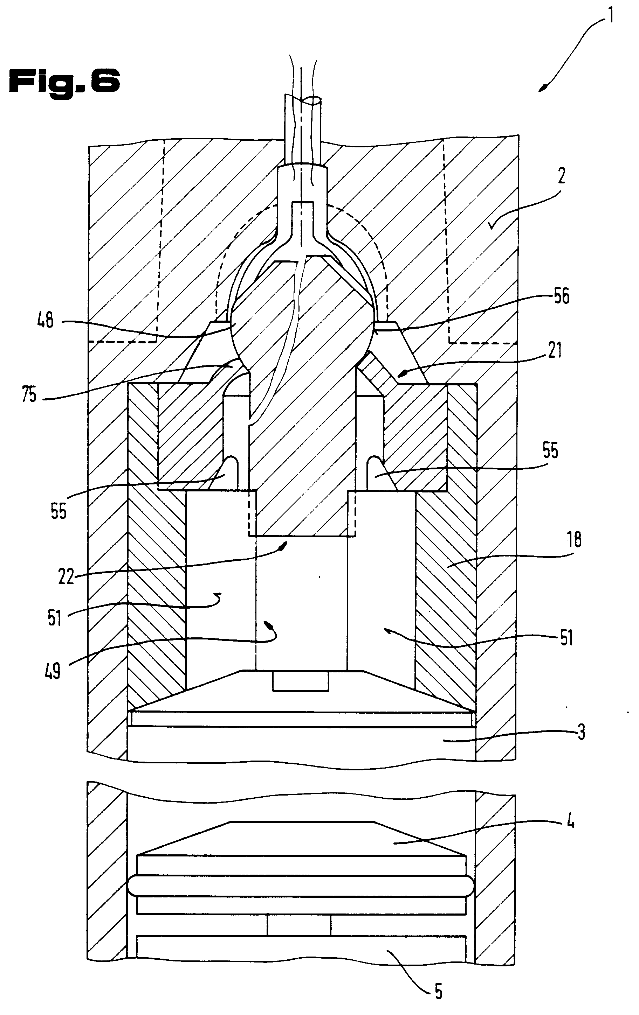

Diese "Dichtstellung" in der Sperrstellung des Umschaltorgans 22, in welcher die Oberfläche 56 an der Stirnfläche 75 der Dichtungsmembran anliegt ist in Fig.6 gezeigt.

In der linken Hälfte dieser Darstellung in Fig.6 ist weiters auch gezeigt, daß bei übermäßiger Druckbeaufschlagung des Umschaltorgans 22 von der Injektionsnadel 6 her - wie sie z.B. bei einer Druckbefüllung auftreten kann - durch die Verwendung eines Elastomers für die Herstellung der Dichtungsmembran bzw. des Stützrings 26 sich der Stützring 26 stulpenförmig verformt und damit eine zur Längsachse 28 zu gerichtete Vorspannkraft in der Dichtungsmembran aufgebaut wird, die zu einem noch festeren dichtenden Abschluß zwischen der Dichtungsmembran bzw. der Stirnfläche 75 und dem Übergangsbereich von dem Führungszapfen 50 zu dem Dichtabschnitt 48 führt.

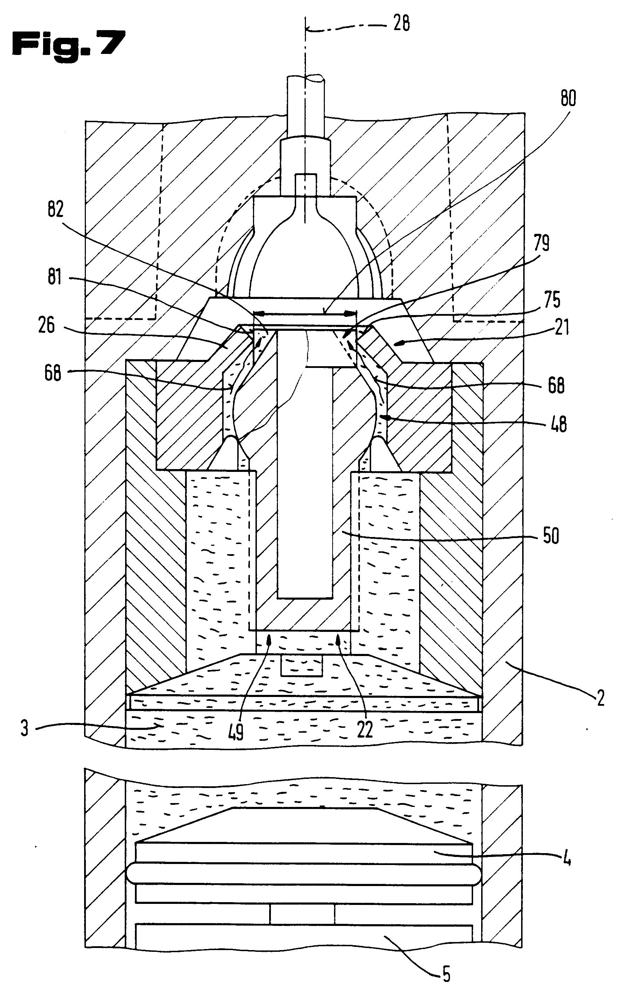

In Fig.7 ist eine weitere Ausführungsvariante für das Umschaltorgan 22 bzw. den Führungszapfen 50 gezeigt. Bei diesem ist im Anschluß an den Dichtabschnitt 48 ein Dichtansatz 79 vorgesehen, der einen Außendurchmesser 80 aufweist, der geringfügig größer ist, als der Innendurchmesser 34 der Öffnung 33 in dem als Dichtungsmembran wirkenden Stützring 26, wie er im entspannten Zustand in Fig.2 gezeigt ist. Dies bewirkt, daß die Öffnung 33, wie mit vollen Linien gezeichnet, geringfügig gegenüber der in strichlierten Linien in Fig.7 und in vollen Linien in Fig.2 dargestellten Ruhestellung aufgeweitet ist und die Stirnfläche 75 mit ihrer Innenkante dichtend an einer Außenumfangsfläche 81 des Dichtansatzes 79 anliegt. Dadurch wird in Verbindung mit der Führung des Führungszapfen 50 in der Führungsbahn 49 auch während der Lagerung und des Transportes eine zentrische Unterstützung des aus einem Elastomer bzw. leicht verformbaren Materials bestehenden Stützrings 26 sichergestellt.

Diese Ausrichtung der Dichtungsmembran bzw. der dichte Abschluß zwischen dem Stützring 26 bzw. dem Dichtansatz 79 bewirkt aber weiters, daß beim Auspressen des Injektionsmediums aus dem Innenraum 3 des Spritzenzylinders 2 mittels des Kolbens 4 über die Kolbenstange 5 bedingt durch die geringe Durchtrittsfläche und entsprechend den Pfeilen 67' eine zusätzliche Vorschubkraft auf das Umschaltorgan 22 ausgeübt wird, sodaß beim Auspressen des Mediums 53 ein zuverlässiges Umschalten des Umschaltorgans 22 aus der in vollen Linien gezeigten Ruhestellung in die in strichlierten Linien gezeichnete Sperrstellung erreicht wird.

Um das Aufziehen des Injektionsmediums zu ermöglichen ist jedoch dabei in der Außenumfangsfläche 81 zumindest ein parallel zur Längsachse 28 verlaufender Durchlaßkanal 82 anzuordnen, sodaß beim Aufziehen des Injektionsmediums in den Innenraum 3 dieses ohne Probleme einströmen kann.

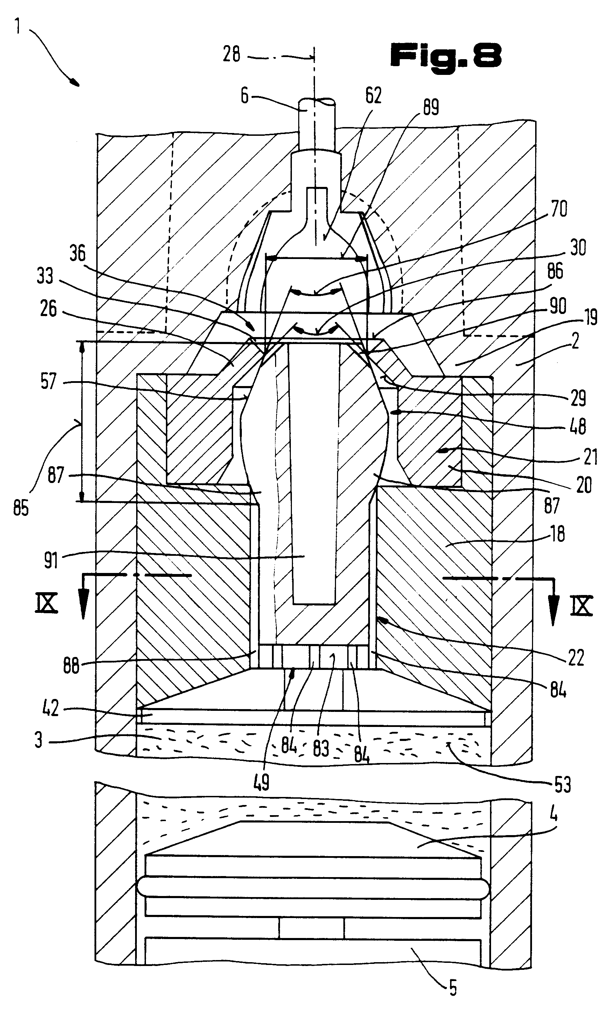

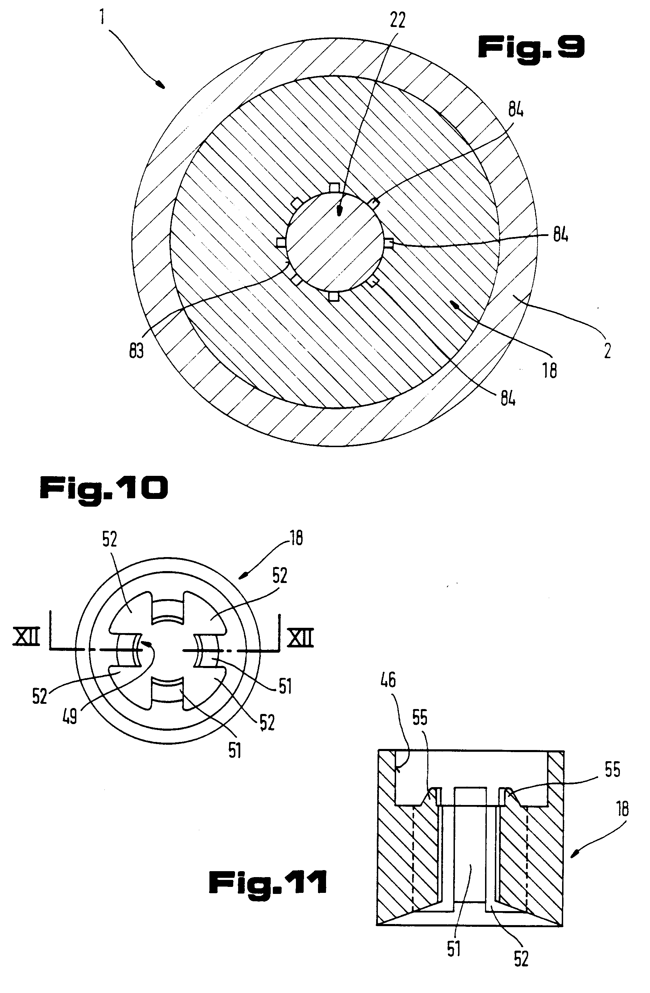

In Fig.8 und 9 ist eine Ausführungsvariante der Injektionseinrichtung 1 gezeigt, wobei alle jene Teile, die mit der in den im Ausführungsbeispiel gemäß den Fig.1 bis 7 beschriebenen übereinstimmen, mit den gleichen Bezugsziffern bezeichnet werden.

Die Injektionseinrichtung 1 umfaßt wiederum einen Spritzenzylinder 2, in deren Innenraum 3 ein Kolben 4 mittels einer Kolbenstange 5 verschiebbar ist. Zwischen Ansätzen 19 und dem Anschlagteil 42 ist ein Gehäuse 18 zur Halterung eines Rückhaltegliedes 21 dargestellt, das wie in den zuvor beschriebenen Ausführungsvarianten durch eine Scheibe 20 aus einem Elastomer, also aus einem elastisch dehn- und rückstellbaren Material, insbesondere einem Kunststoff oder einem Pharmagummi oder Silikonkautschuk, insbesondere mit einer Härte von Shore A 40 bis 65 hergestellt ist.

Das Gehäuse 18 ist wiederum aus einem harten bei Raumtemperatur steifen und führungsgenauen Kunststoff hergestellt.

Ein als Dichtmembran dienender Stützring 26 ist in einen Freiraum 36 frei verformbar um einen Durchtritt eines Umschaltorganes 22 aus der in vollen Linien gezeichneten Ruhestellung in eine im Freiraum 36 bzw. einer Aufnahmekammer 62 befindliche Sperrstellung zu ermöglichen.

Gegenüber den zuvor beschriebenen Ausführungsvarianten ist das Umschaltorgan 22 im Bereich des Dichtabschnittes 48 anders ausgebildet. Das Umschaltorgan 22 ist mit seinem Führungselement 23 in einer durch eine Führungsbohrung 83 gebildeten Führungsbahn 49 exakt längsverschiebbar geführt gelagert. Die Toleranzen zwischen dem Führungselement 23 und der Führungsbahn 49 sind sehr gering um ein Verkanten bzw. Kippen des Umschaltorgans 22 in eine schräg zur Längsachse 28 befindliche Lage zu verhindern. Über den Umfang der Führungsbohrung 83 verteilt sind durch Nuten gebildete Kanäle 84 zum Durchströmen des Mediums 53 vom Innenraum 3 in die Injektionsnadel 6 bzw. von der Injektionsnadel 6 in den Innenraum 3 angeordnet.

Diese Kanäle 84 können mit einer Teilung von 90°, aber mit jeder beliebigen anderen Teilung ebenfalls angeordnet sein, sodaß je nach der durchzuströmenden Flüssigkeitsmenge ein ausreichender Durchtrittsquerschnitt für das Medium 53 vorhanden ist.

Um eine ständig zentrierte Halterung der elastisch verformbaren weichen Dichtungsmembran, also den Stützring 26 zu ermöglichen, weist der Dichtabschnitt 48 eine Länge 85 auf, die in etwa der Distanz zwischen dem Ende der Führungsbahn 49 und einer der Injektionsnadel 6 zugewandten Stirnkante 86 des Stützringes 26 entspricht.

Vor allem wird der Öffnungswinkel 70 des die Oberfläche 57 bildenden Drehkegelabschnittes des Dichtabschnittes 48 kleiner gewählt, als ein Öffnungswinkel 30 der Innenflächen 29 des Stützringes 26.

Weiters ist dabei zu berücksichtigen, daß bei Anlage von Halterippen 87 im Übergangsbereich zwischen den Dichtabschnitt 48 und dem Führungszapfen 50 angeordnet sind und bevorzugt auf den zwischen den Kanälen 84 verbleibenden Stegen 88 aufliegen, ein Durchmesser 89 des drehkegelstumpfförmigen Dichtabschnittes 48 dem Durchmesser 34 des Stützringes 26 im Ruhezustand - wie beispielsweise in Fig.2 gezeigt ist -, entspricht. Damit wird eine dichtende Anlage des Dichtabschnittes an dem die Dichtungsmembran bildenden Stützringes 26 bzw. an dessen Innenkante erreicht. Um bei Erzeugung eines Unterdruckes im Innenraum 3 zum Aufziehen des Mediums 53 in den Innenraum 3 beispielsweise mit dem Kolben 4 ein Durchfließen des Mediums zwischen dem Umschaltorgan 22 und der Dichtungsmembran zu ermöglichen, können am Umfang des Dichtabschnittes ein oder mehrere Durchtrittskanäle 90 vorgesehen werden, die auch gleichzeitig zum Entlüften verwendet werden können.

In jedem Fall ist jedoch darauf zu achten, daß der Querschnitt des Durchtrittskanals 90 bzw. die Summe der Querschnitte nicht so hoch wird, daß durch diese so viel Medium 53, beispielsweise beim Auspressen desselben mittels des Kolben 4 zwischen dem Stützring 26 und dem Umschaltorgan 22 hindurchtreten kann, daß der verbleibende Druckmitteldruck nicht mehr ausreicht, um das Umschaltorgan 22 durch den Stützring 26 in die Sperrstellung hindurchzudrücken.

Der Vorteil dieser Lösung liegt darin, daß eine Druckbefüllung zusätzlich erschwert wird, da die Injektionseinrichtung 1 auch im nicht benutztem Zustand einen dichtenden Abschluß zwischen Injektionsnadel 6 und Innenraum 3 aufweist und darüber hinaus die Öffnung 33 der Membran bzw. des Stützringes 26 immer exakt auf die Längsachse 28 zentriert ist.

Wie aus dieser Darstellung weiters zu ersehen ist, können für die Positionierung des Umschaltorgans 22 in Bezug auf die Lage in Richtung der Längsachse 28 vor allem in der Ruhestellung oder beim Aufziehen des Mediums 53 anstelle der Führungsnasen 55 die Halterippen 87 vorgesehen sein, um einen ausreichenden Durchtrittsquerschnitt für den Durchtritt des Mediums 53 zwischen dem Dichtabschnitt 48 und der Scheibe 20 in die Kanäle 84 sicherzustellen.

Aus Fig.9 ist ersichtlich, daß die Kanäle 84 über den gesamten Umfang der Führungsbohrung 83 angeordnet sind. Das Umschaltorgan 22 kann, insbesondere auch aus Gründen der Herstellung im Spritzgußverfahren mit einer Kernausnehmung 91 versehen sein, um aufgrund der dadurch bewirkten rohrförmigen Gestalt des Umschaltorgans eine höhere Steifigkeit um gleichzeitig ein besseres Kühlverhalten des Spritzgußteils zu erzielen.

Ein weiterer Vorteil der Verwendung eines harten Kunststoffes für das Gehäuse 18 und das Umschaltorgan 22 liegt vor allem auch darin, daß der Reibungswiderstand derartiger harter Kunststoffteile erheblich geringer ist und daher die aufzubringende Kraft zur Verstellung des Umschaltorgans 22 aus der Ruhestellung in die Sperrstellung unabhängig vom Verformungswiderstand des Stützringes bzw. der Dichtungsmembran gering gehalten werden kann. Vor allem liegt ein wesentlicher Vorteil der beschriebenen Ausführungsvarianten für das Umschaltorgan 22 darin. daß dieses nur im Bereich des Durchtrittes durch den als Dichtungsmembran wirkenden Stützring 26 in Reibungsschluß mit dem Elastomer, also mit dem elastisch leicht verformbaren Material steht und in den übrigen Bereichen auch über die an den Stützring 26 anschließenden Bereiche der Scheibe 20 von diesen freigestellt ist, sodaß auch hier keine unnötige Reibkrafterhöhung bzw. ein Reibwiderstand entstehen kann.

Vor allem ist es dann vorteilhaft für das Gehäuse 18, unabhängig davon, ob dies aus einem oder mehreren Gehäuseteilen besteht bzw. für das Umschaltorgan 22 Kunststoffe mit guten Gleiteigenschaften zu verwenden, sodaß der Kraftaufwand zur Aktivierung des Umschaltorgans 22 zur Verstellung aus der Ruhestellung in die Sperrstellung möglichst gering gehalten werden kann und mit den üblicher Weise bei der Betätigung von Injektionsspritzen teilweise auch durch Normen vorgegebenen Betätigungskräften im Bereich von 5 N bis 10 N das Auslangen gefunden werden kann.

In Fig. 10 und 11 ist das Gehäuse 18 als Einzelteil dargestellt. Aus dieser Darstellung ist die exakte Ausbildung der Führungsstege 51 und der Kanäle 52 zu entnehmen. Dadurch, daß über die Kanäle 52 eine breitflächige tangentiale Anströmung der kugelkalottenförmig ausgebildeten Oberfläche 56 erzielt wird, wird das Durchtreiben des Umschaltorgans bei einer Ausübung eines Druckes auf das Medium 53 über den Kolben 4 begünstigt. Gleichzeitig wird aber durch die präzise Ausführung der Führungsbahn 49 im Bereich der Führungsstege 51 eine präzise und kippsichere Führung des Umschaltorgans 22 sichergestellt.

Des weiteren ist insbesondere in Fig.11 die Ausnehmung 46, in welche das Rückhalteglied 21 eingesetzt werden kann, sowie die Führungsnasen 55 zum Zentrieren der Scheibe 20 des Rückhaltegliedes 21 sowie zum Positionieren des Umschaltorganes 22 in der Ruhestellung ersichtlich.

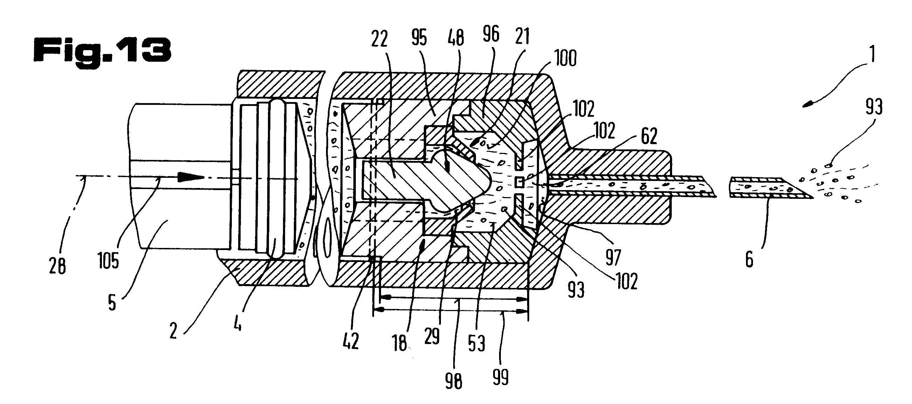

In den Fig.12 bis 19 ist eine andere Ausführungsvariante einer erfindungsgemäßen Injektionseinrichtung 1 dargestellt. Die Fig. 13 bis 16 unterscheiden sich von der Fig. 12 nur dadurch, daß in Fig. 12 die Stellung der Originalitätssicherungsvorrichtung 7 beim Aufziehen eines Mediums 53 aus einer Ampulle 92 gezeigt ist, während in Fig.13 die Stellung zum Entlüften, also zum Ausbringen von Luftblasen 93 aus dem Medium vor dem Injizieren des Mediums 53 in den Körper eines Patienten dargestellt ist.

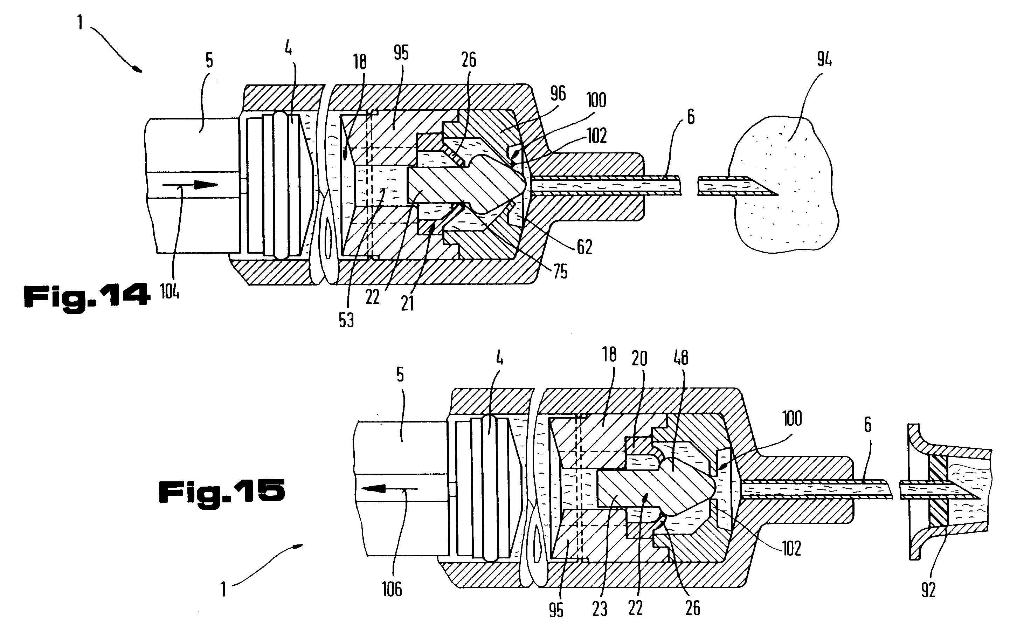

Die Fig.14 zeigt die Stellung der Originalitätssicherungsvorrichtung 7 beim Auspressen des Mediums 53 in einen Körper 94 eines Patienten, während Fig.15 die Stellung der Originalitätssicherungsvorrichtung 7 bei dem Versuch während des Auspressens des Injektionsmediums ein weiteres Injektionsmedium aus einer Ampulle 92 aufzuziehen, zeigt.

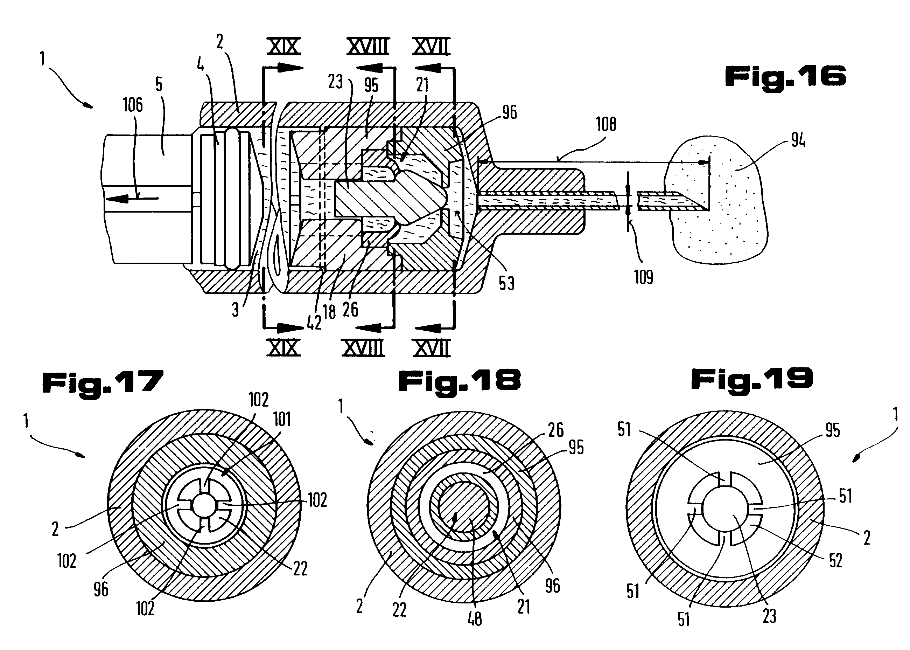

In Fig.16 ist weiters eine Möglichkeit dargestellt, wie unabhängig von einer ausreichenden Elastizität der Dichtungsmembran bzw. des Stützrings 26 mit der in den Fig.12 bis 19 dargestellten Injektionseinrichtung 1 aspiriert werden kann.

Nachdem eine Vielzahl der Teile der vorliegenden Injektionseinrichtung 1 mit den Teilen in den zuvor beschriebenen Injektionseinrichtungen 1 übereinstimmen, werden bei dieser nun zu beschreibenden Injektionseinrichtung 1 für gleichartige Teile die gleichen Bezugsziffern wie vorher verwendet. Die Injektionseinrichtung 1 besteht wiederum aus einem Spritzenzylinder 2 mit einem Innenraum 3, in dem das Medium 53 zum Injizieren zwischen einem Kolben 4, der mit einer Kolbenstange 5 verbunden ist, und einer Injektionsnadel 6 angeordnet ist.

Zwischen der Injektionsnadel 6 und dem Innenraum 3 des Spritzenzylinders 2 befindet sich eine Originalitätssicherungsvorrichtung 7, die wiederum ein Umschaltorgan 22 aufweist, welches die verschiedenen in den Fig.12 bis 16 gezeigten Stellungen einnehmen kann.

Im Unterschied zu den zuvor beschriebenen Ausführungsbeispielen besteht jedoch das Gehäuse 18 aus einem Gehäuseteil 95 und einem Gehäuseteil 96, ist also zweiteilig ausgeführt. Das Rückhalteglied 21, welches in gleicher Weise, wie zuvor beschrieben aufgebaut sein kann, umfaßt wiederum eine Scheibe 20 und den als Dichtungsmembran wirkenden Stützring 26.

Das Gehäuse 18 ist in den Spritzenzylinder 2 in dem der Injektionsnadel 6 zugewandten Auslaß- bzw. Einlaßbereich 37 angeordnet und kann beispielsweise bei aus Kunststoff bestehenden Injektionszylindern mit diesen verschweißt bzw. verklebt sein oder zwischen einer Stirnwand 97 des Spritzenzylinders 2 und einem Anschlagteil 42, der durch einen über eine Innenfläche 43 des Spritzenzylinders 2 vorspringenden Bund 44 gebildet sein kann, befestigt sein.

Die das Gehäuse 18 bildenden Gehäuseteile 95, 96 bzw. das Rückhalteglied 21 können in einem Verbindungsbereich zur Erzielung einer montagefertigen Baugruppe miteinander nach den Einsetzen des Rückhaltegliedes 21 und gegebenenfalls des Umschaltorgans 22 verschweißt bzw. verklebt werden. Möglich ist aber auch, daß die Gehäuseteile 95, 96 bzw. das Rückhalteglied 21 über Schnapp- und/oder Rastverbindungen miteinander verbindbar ausgebildet sind.

So ist es beispielsweise, wie in Fig.13 gezeigt, auch möglich, daß eine Führungslänge 98 des Gehäuses 18 kleiner ist als eine Distanz 99 zwischen der Stirnwand 97 und dem Anschlagteil 42.

Die genaue Funktion dieses Unterschiedes zwischen der Führungslänge 98 und der Distanz 99 wird dann anhand der Darstellung in Fig.16 näher erläutert werden.

Der Gehäuseteil 96, welcher zwischen dem Rückhalteglied 21 und der Stirnwand 97 des Spritzenzylinders 2 angeordnet ist, weist den zum Ausdehnen der Dichtungsmembran, insbesondere des Stützrings 26 benötigten Freiraum 36 auf, der in Richtung der Injektionsnadel 6 als Rückstellvorrichtung 100 ausgebildet ist. Diese Rückstellvorrichtung 100 umfaßt im Abstand über den Umfang einer Durchtrittsöffnung 101 verteilte Rückstellarme 102 auf, die als Federarme ausgebildet und elastisch verformbar sein können. Durch das dem jeweils gewählten Kunststoffmaterial entsprechende Rückstellverhalten bzw. dem sogenannten Memory-Effekt wird nach jeder Verformung der Rückstellarme 102 in Richtung der Injektionsnadel 6 durch das über den Flüssigkeitsdruck erfolgte Vordrücken des Umschaltorgans 22 eine entsprechende Rückstellfederkraft aufgebaut, die unmittelbar nach dem Unterbrechen der Druckkraft zu einer Rückstellung des Umschaltorgans 22 in die Ausgangslage bzw. in eine am Stützring 26 anliegende, dichtende Stellung bewirkt.

Zwischen den Rückstellarmen 102 und der Injektionsnadel 6 kann weiters eine Aufnahmekammer 62 der Stirnwand 97 vorgeordnet sein, sodaß ein entsprechender Freiraum zur elastischen Verformung der Rückstellarme 102 vorhanden ist.

Während nun in Fig.12 gezeigt ist, wie das Medium 53 aus einer Ampulle 92 aufgezogen werden kann, ist in der Fig.13 das Entlüften der Injektionseinrichtung 1 gezeigt. Um eine frühzeitige Auslösung bzw. Betätigung des Umschaltorgans 22 zu verhindern ist in diesem Fall auf einer Innenfläche 29 des Rückhaltegliedes 21 eine beispielsweise durch eine Noppe gebildete Erhöhung 103 in Form einer Warze eines länglichen, beispielsweise parallel zur Längsachse 28 verlaufenden Bundes oder dgl. vorgesehen. Wird nun ein gegenüber dem Auspressen des Injektionsmediums geringerer Druck, der gegenüber dem vollen Auspreßdruck, wie in Fig.14 durch einen dicken Pfeil 104 symbolisiert ist, durch einen dünneren Pfeil 105 gezeigt ist, auf das Injektionsmedium ausgeübt und wird der Spritzenzylinder 2 vibriert bzw. die Injektionseinrichtung 1 geschüttelt, so treten die im Medium 53 enthaltenen Luftblasen 93, wie schematisch angedeutet, durch die Injektionsnadel 6 nach Außen, da entgegen der Flüssigkeit für die der Durchtrittsquerschnitt zwischen dem Stützring 26 und dem Umschaltorgan 22 im Bereich des Dichtabschnittes 48 zu gering ist, die Lufteinschlüsse bzw. Luftblasen 93 hindurchtreten können.

Wenn dann das Medium 53 in der Injektionseinrichtung 1 entlüftet ist, kann durch Ausüben der vollen Vorschubkraft die entsprechend den normgemäßen Vorgaben maximal zwischen 7,5 N bis 10 N betragen soll, das Medium 53 in den Körper 94 eines Patienten injiziert werden. Durch die über das Medium 53 über den Kolben 4 und die Kolbenstange 5 vom Bediener ausgeübte Druckkraft wird das Umschaltorgan 22 in die in Fig.14 gezeigte Stellung durch den als Dichtungsmembran wirkenden Stützring 26 hindurchgedrückt, bis er an den Rückstellarmen 102 der Rückstellvorrichtung 100 anliegt, wobei bei entsprechend hoher Druckkraft diese Rückstellarme 102 elastisch unter Aufbau einer Rückstellkraft verformt werden.

Sobald eine Druckkraft gemäß Pfeil 104 auf den Kolben 4 ausgeübt wird, bläht sich der als Dichtungsmembran wirkende Stützring 26 auf und das Medium 53 kann zwischen dem Umschaltorgan 22 und dem Stützring 26 des Rückhaltegliedes 21 und zwischen den Rückstellarmen 102 in die Injektionsnadel 6 und von dort in den Körper 94 des Patienten eintreten.

Bei jeder Unterbrechung des Injektionsvorganges wird durch die Rückstellarme 102 bzw. die in diesen aufgebaute Rückstellkraft das Umschaltorgan 22 entgegen der Wirkung der Auspreßkraft zurück gegen den Stützring 26 bzw. dessen umlaufende Stirnfläche 75 gepreßt. Dadurch wird jederzeit eine gesicherte Abdichtung des Innenraumes 3 von Außen her sichergestellt, sodaß das Aufziehen oder Einpressen eines weiteren Mediums nach der erstmaligen Abgabe eines Mediums 53 zuverlässig verhindert ist.

In Fig.15 ist gezeigt, daß durch die in Richtung der Injektionsnadel 6 sich verjüngende Ausbildung des Stützringes 26 sich dieser auf der dem Führungselement 23 zugewandten Seite des Umschaltorgans 22 bzw. des Dichtabschnittes 48 festsetzt und durch die Rückstellarme 102 eine so große Rückstellkraft im Sinne eines Pfeils 106 ausgeübt wird, daß ein dichter Abschluß zwischen dem Dichtabschnitt 48 und dem Stützring 26 entsteht.

Schlußendlich ist in Fig.16 dargestellt, wie mit der beschriebenen Injektionseinrichtung 1 aspiriert werden kann. Wird nämlich zuerst eine geringe Menge an Medium 53 in einen Körper 94 eines Patienten verabreicht, so kann dann durch Zurückziehen der Kolbenstange 5 im Sinne des Pfeiles 106 mit dem Kolben 4 ein Unterdruck im Innenraum 3 des Spritzenzylinders 2 aufgebaut werden. Dieser bewirkt aufgrund der geringeren Führungslänge 98 gegenüber der Distanz 99, daß sich das gesamte Gehäuse 18 bestehend aus den beiden Gehäuseteilen 95 und 96, die entweder miteinander verklebt oder durch einen Schweißvorgang oder eine Schnapp- oder Preßverbindung miteinander verbunden sein können, über die Differenz zwischen der Führungslänge 98 und der Distanz 99 in Richtung des Kolben 4 bis zur Anlage am Anschlagteil 42 zurückgezogen wird.

Das sich durch den Hub 107 und den Flächenquerschnitt im Innenraum 3 des Spritzenzylinders 2 ergebende Volumen kann nun dazu genutzt werden, um zumindest ein sich aus einer Nadellänge 108 und einem Innendurchmesser 109 der Injektionsnadel 6 ergebendes Volumen in den Spritzenzylinder 2 aufzusaugen, wobei bevorzugt das aufzusaugende Volumen zumindest geringfügig größer, als das sich aus dem Nadelvolumen ergebende Volumen ist.

Wird nun nach dem Injizieren einer geringen Menge des Injektionsmediums der Kolben 4 bis zur Anlage des Gehäuses 18 am Anschlagteil 42 zurückgezogen, kann festgestellt werden, ob in der zurückgezogenen Flüssigkeitsmenge Blut enthalten ist, sodaß ein ordnungsgemäßer Aspiriervorgang durchgeführt werden kann.

Wesentlich ist dabei, daß während dieses Aspiriervorganges die Originalitätssicherungsvorrichtung 7 nicht ausgelöst wird, sodaß gegebenenfalls dann, wenn der Arzt beim Einstechen eine Vene getroffen hat, der Aspiriervorgang nochmals wiederholt werden kann, bevor das Medium 53 vollständig in den Körper 94 des Patienten abgegeben wird.

Wesentlich ist hierbei, daß es durch eine derartige mögliche Ausbildung die jedoch keinesfalls zwingend ist, auch möglich ist, den Aspiriervorgang dann durchzuführen, wenn die Originalitätssicherungsvorrichtung 7 ausgelöst und das Umschaltorgan 22 von der Ruhe- in die Sperrstellung verstellt wurde. Trotzdem ist aber sichergestellt, daß während des Aspiriervorganges durch die Abdichtung des Umschaltorganes 22 kein Medium 53 in den Innenraum 3 des Spritzenzylinders 2 aufgezogen werden kann.

In den Fig.17 bis 19 sind Schnitte durch verschiedene Bereiche der Injektionseinrichtung 1 dargestellt.

So ist in Fig.17 die exakte Anordnung und Verteilung der Rückstellarme 102 der Rückstellvorrichtung 100 gezeigt. Beim vorliegenden Ausführungsbeispiel werden vier über den Umfang verteilte Rückstellarme 102 eingesetzt. Es ist jedoch auch möglich, mit nur einem oder zwei Rückstellarmen das Auslangen zu finden, oder auch mehr als vier Rückstellarme vorzusehen.

Die Darstellung in Fig.18 zeigt die Überschneidung und Anordnung der Gehäuseteile 95 und 96 sowie des Stützringes 26 des Rückhaltegliedes 21 im Anlagebereich des die Dichtungsmembran bildenden Stützringes 26 am Dichtabschnitt 48 des Umschaltorganes 22.

Aus Fig.19 ist zu ersehen, daß das Führungselement 23 zwischen Führungsstegen 51 des Gehäuseteils 95 geführt ist, die zwischen sich Kanäle 52 zum Durchtritt des Mediums 53 parallel zum Führungselement 23 bilden, sodaß auch eine Tangentialeinströmung des über den Umfang des Führungselementes 23 hinausragenden Querschnittes des Dichtabschnittes 48 möglich ist.

Die Anordnung und räumliche Ausbildung des Dichtabschnittes 48 kann in Anpassung an die gewünschte Ausführungsvariante im Rahmen der zuvor beschriebenen Ausführungsvarianten und der nachstehend beschriebenen Ausführungsvarianten beliebig verändert werden. Vor allem ist es auch möglich, daß anstelle der Kugelkalottenausbildung im Bereich der dem Führungselement 23 zugewandten Oberfläche des Umschaltorganes 22 auch eine drehkegelförmige Ausbildung möglich ist.

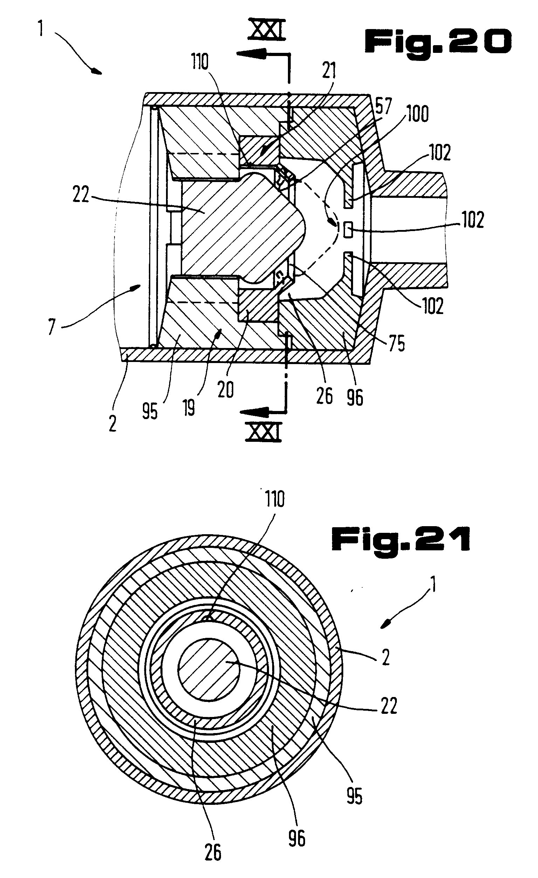

In den Fig.20 und 21 ist ein Teil der Injektionsvorrichtung nach den Fig.12 bis 19 in größerem Maßstab mit geringfügigen Änderungen gegenüber der zuvor beschriebenen Ausführungsform dargestellt.

Vor allem in Fig.20 ist nur der Teil der Injektionseinrichtung 1 gezeigt, in welchem die Originalitätssicherungsvorrichtung 7 angeordnet ist. Es ist daraus zu ersehen, daß das Gehäuse 18 aus zwei Gehäuseteilen 95, 96 zusammengesetzt ist, zwischen welchen das Rückhalteglied 21, insbesondere die Scheibe 20 mit dem Stützring 26 angeordnet ist. Der Gehäuseteil 95 umfaßt als integrierten Bauteil auch die Rückstellvorrichtung 100 mit den Rückstellarmen 102, die in Bewegungsrichtung des Umschaltorganes 22 elastisch verformbar sind.

Um nun bei einer derartigen Ausführungsform dann, wenn das Umschaltorgan 22 mit seiner Oberfläche 57 am Stützring 26 anliegt, ein Entlüften des im Innenraum 3 des Spritzenzylinders 2 vorhandenen Mediums 53 zu ermöglichen, ist nunmehr über die Bauhöhe des Rückhaltegliedes 21 ein Entlüftungskanal 110 angeordnet.

Für die Auslegung dieses Entlüftungskanals 110, es können selbstverständlich auch mehrere derartige Kanäle über den Umfang des Rückhaltegliedes 21 verteilt angeordnet sein, gelten dieselben Kriterien, wie für die Anordnung der in Fig.2 beschriebenen Entlüftungskanäle 60. Sie müssen von ihrem Querschnitt bzw. von ihrem Verlauf derart ausgebildet sein, daß sie lediglich einen Durchtritt von Luft aber keinesfalls einen Durchtritt vom Medium 53 ermöglichen.

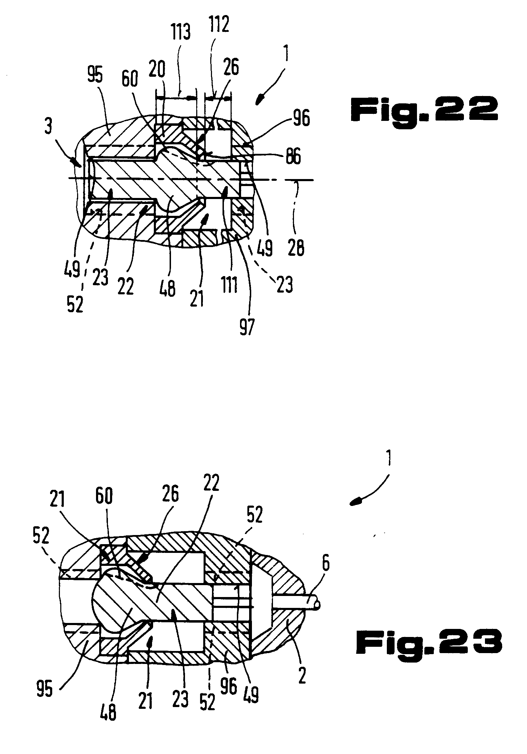

In Fig.22 und 23 sind dann noch weitere Ausführungsvarianten für die Führung des Umschaltorganes 22 und die Anordnung der Führungsbahn 49 gezeigt.

So ist bei dem Ausführungsbeispiel in Fig.22 der Dichtabschnitt 48 des Umschaltorganes 22 im Bereich beider Stirnenden mit einem Führungselement 23 und 111 verbunden.

Dadurch, daß nunmehr auf beiden Seiten des Dichtabschnittes 48 Führungselemente 23 und 111 vorgesehen sind und beiden dieser Führungselemente 23 und 111 im jeweiligen Gehäuseteil 95 und 96 eine eigene Führungsbahn 49 zugeordnet ist, ist eine exakte zentrische Bewegung des Umschaltorgans 22 entlang bzw. parallel zur Längsachse 28 möglich. Dadurch kann auch mit relativ kurzen Führungslängen für die Führungselemente 23 und 111 das Auslangen gefunden werden, da ein Kippen des Umschaltorgans 22 vor allem während des Durchtrittes durch das Rückhalteglied 21 verhindert ist. Um eine einwandfreie Funktion und einen sicheren Durchtritt des Dichtabschnittes 48 durch das Rückhalteglied 21 zu ermöglichen, ist eine Distanz 112 gleich oder größer einem Abstand 113 zu wählen. Der Abstand 113 erstreckt sich dabei zwischen dem Übergangsbereich zwischen dem Führungselement 23 und dem Dichtabschnitt 48 einerseits und dem Übergangsbereich zwischen dem Dichtabschnitt 48 und dem Führungselement 111 andererseits. Sowohl die Distanz 112 als auch der Abstand 113 werden parallel zur Längsachse 28 der Injektionseinrichtung 1 ermittelt. Um ein ungehindertes Vorbeiströmen des Mediums 53 an den Führungselementen 23 und 111 zu ermöglichen, können wiederum Kanäle 52 im Bereich des Führungselementes 23 bzw. etwa gleich angeordnete Kanäle im Bereich des Führungselementes 111 angeordnet sein.

Um auch bei dieser Injektionseinrichtung 1 gemäß Fig.22 eine einwandfreie Entlüftung des Innenraumes 3 des Spritzenzylinders 2 zu ermöglichen, kann im Bereich des Dichtabschnittes 48 bis in den Bereich des Führungselementes 111 ein Entlüftungskanal 60 angeordnet sein.

Die in Fig.23 gezeigte Ausführungsvariante unterscheidet sich von der in Fig.22 gezeigten Variante nur dadurch, daß ausschließlich in dem Gehäuseteil 96, also demjenigen, der der Injektionsnadel 6 näher liegend angeordnet ist, eine Führungsbahn 49 angeordnet ist und sich nur der Dichtabschnitt 48 des Umschaltorgans 22 in der Ruhestellung jenseits des Stützringes 26 des Rückhaltegliedes 21 befindet. Diese Ausführungsform hat den Vorteil, daß die gesamte Fläche des Dichtabschnittes auf der dem Kolben 4 zugewandten Seite zum Ausüben einer Druckkraft zum Durchschieben des Dichtabschnittes beim Umschalten von einer Ruhe- in eine Sperrstellung zur Verfügung steht und damit die Sicherheit zum Auslösen des Umschaltorganes 22 erhöht werden kann.

Vorteilhaft ist bei dieser Ausgestaltung aber auch, daß das Dichtelement, also der Stützring 26 des Rückhaltegliedes 21 während der gesamten Dauer der Verwendung der Injektionseinrichtung 1 sich zentriert auf dem Führungselement 111 abstützen kann, wodurch auch in den verschiedenen Positionen ein entsprechend dichter Abschluß zwischen dem Stützring 26 und dem zugeordneten Führungselement 23, 111 bzw. dem Dichtabschnitt 48 erreicht wird. Selbstverständlich kann auch bei dieser Ausführungsvariante der Injektionseinrichtung 1 im Bereich des Stützring 26 ein Entlüftungskanal 60 in der Umfangsfläche des Dichtabschnittes 48 angeordnet sein.

Bevorzugt soll eine Überdeckung des Führungselementes 23, 111 und der Führungsbahn 49, vor allem, wenn nur auf einer Seite des Dichtabschnittes ein Führungselement 23 bzw. 111 angeordnet ist, zwischen 0,8 bis 1,5 mal den Durchmesser des Führungselementes 23, 111 betragen. Grundsätzlich ist jedoch die Mindestüberdeckung zwischen dem Führungselement 23 bzw. 111 und der Führungsbahn 49 in Abhängigkeit von der maximalen Kipptoleranz bzw. dem maximal erlaubten Kippwinkel festzulegen. Je geringer die Toleranz beim Kippwinkel ist, um so länger ist die Überdeckung zwischen dem Führungselement 23 bzw. 111 und der Führungsbahn 49 zu wählen. Dazu kommt jedoch, daß bei geringerer Maßtoleranz in den Durchmesserabmessungen der Führungselemente 23 bzw. 111 und der Führungsbahn 49 diese Überdeckungslänge geringer gewählt werden kann, als bei höheren Toleranzen d.h. ungenaueren Fassungen. Vorteilhaft ist dabei, wenn das Spiel zwischen dem Führungselement 23 bzw. 111 und der Führungsbahn 49 im Bereich von 0,01 mm und 0,05 mm, also eine Passung bzw. Feinpassung mit eingeschränktem Toleranzbereich ist, um zuverlässig ein Kippen oder Schrägstellen des Umschaltorgans 22 in der Führungsbahn 49 zu verhindern.

Grundsätzlich ist bei allen den zuvor beschriebenen Ausführungsbeispielen zu berücksichtigen, daß die im Bereich der Führungsbahn 49 angeordneten Kanäle 52 bzw. 84 bei einem bevorzugten Ausführungsbeispiel in Summe eine Querschnittsfläche aufweisen, die gleich oder kleiner der Querschnittsfläche der dem Kolben 4 zugewandten Stirnseite 68 des Umschaltorgans 22 ist.

Je größer nämlich die Querschnittsfläche der Kanäle 52 bzw. 84 ist, umso höher ist der Druckverlust der beim Durchströmen des Injektionsmediums durch diese Kanäle 52 bzw. 84 auftritt und das kann dann vor allem bei niedrig viskosen Flüssigkeiten dazu führen, daß der auf die Stirnseite 68 ausgeübte Druck nicht mehr ausreicht um das Umschaltorgan 22 durch das Rückhalteglied 21 hindurchzubewegen bzw. die Originalitätssicherungsvorrichtung zu aktivieren, um damit eine weitere Verwendung der Injektionsspritze zu unterbinden.

Dabei ist weiters auch zu berücksichtigen, daß auch bei hoch viskosen Flüssigkeiten bzw. Injektionsmedien der Anteil der seitlich bzw. tangential das Umschaltorgan 22 anströmenden Flüssigkeitsmenge nicht zu hoch sein soll, da diese Quereinströmung die Vorwärtsbewegung des Umschaltorgans 22 in Richtung der Injektionsnadel beim Auspressen des Injektionsmediums behindert und verzögert und daher ebenfalls der gewünschten Umschaltbewegung entgegenwirken kann.

Aus diesem Grund ist es daher vorteilhaft, wenn die Gesamtquerschnittsflächen aller Kanäle 52 bzw. 84 nur etwa zwischen 40 und 70 % der Querschnittsfläche der Stirnseite 68 betragen.

Des weiteren ist in Ergänzung zu den Ausführungen, betreffend die Ausbildung der Kanäle 60 oder der Durchtrittskanäle 90, unter Bezugnahme auf die bereits vorstehenden Ausführungen, insbesondere zum Ausführungsbeispiel, wie es unter anderem in Fig. 2 gezeigt ist, bzw. zum Ausführungsbeispiel wie es in Fig. 8 gezeigt ist festzuhalten, daß die Querschnittsfläche bzw. der freie Durchtritt durch diese Kanäle 60 bzw. Durchtrittskanäle 90 bei einem bevorzugten Ausführungsbeispiel so gewählt werden sollen, daß ein Flüssigkeitsdurchtritt nicht oder nur in äußerst geringen Mengen möglich ist. Vor allem empfiehlt es sich beispielsweise nur einen solchen Kanal 60 bzw. Durchgangskanal 90 anzuordnen, der sich von der Oberfläche 56 bis in den Bereich einer Stirnfläche 61 des Dichtabschnittes 48 erstreckt. In diesem Fall kann dann dieser Kanal nur zum Auspressen von Luft aus dem Innenraum zwischen dem Umschaltorgan 22 und dem Kolben 4 verwendet werden und es ist ein entsprechender Umgehungskanal für das Aufziehen des Medikamentes durch die Injektionsnadel in diesen Innenraum vorzusehen. Dadurch braucht bei der Querschnittsausbildung und der Querschnittsfläche dieses Kanals auf das Einströmen des Mediums beim Aufziehen keine Rücksicht genommen werden und dieser Kanal 60 kann entsprechend den Forderungen für das Entlüften des Injektionsmediums ausgebildet werden.

Vor allem ist aber bei einem beovorzugten Ausführungsbeispiel darauf zu achten, daß die Querschnittsfläche dieses Kanals nur einen Bruchteil, also einen Bereich von 0,1 bis maximal 10 Prozent der Querschnittsfläche der Stirnseite 68 des Umschaltorgans 22 beträgt, auch wenn diese auf eine Mehrzahl von Kanälen 60 über den Umfang des Umschaltorgans 22 aufgeteilt ist und auch sichergestellt ist, daß keine Flüssigkeit durch diese Kanäle hindurchtreten kann. Der Grund hierfür liegt vor allem darin, daß die durch das Einspressen des Mediums zwischen dem Umschaltorgan 22 und dem Rückhalteglied 21 entstehende Druckkraft nicht größer werden darf, als die zum Durchpressen des Umschaltorgans 22 durch das Rückhalteglied 21 benötigte Kraft, da ansonsten das Umschaltorgan 22 durch den über den Kolben 4 auf das Medium ausgeübten Druck vom Rückhalteglied 21 in Richtung des Kolbens 4 zurückgepreßt und damit der Durchtritt des Injektionsmediums ohne dem Umschalten der Umschaltvorrichtung 22 ermöglicht werden könnte.

Damit empfiehlt es sich für derartige Kanäle nur Querschnitte von 0,005 bis 0,05 mm in Abhängigkeit von der Viskosität des jeweiligen Injektionsmediums zu verwenden, wobei bei höherer Viskosität ein kleinerer Querschnitt zu wählen ist.

Abschließend sei noch darauf hingewiesen, daß selbstverständlich an den erfindungsgemäßen Injektionseinrichtungen jede beliebige Wahl an Kombination und Zusammenstellung der einzelnen Ausbildungsvarianten insbesondere für das Gehäuse 18, das Rückhalteglied 21 und das Umschaltorgan 22 möglich ist, wobei auch die Ausführungsformen derjenigen Teile, die in unterschiedlichen Ausführungsbeispielen beschrieben sind, zu einer Injektionseinrichtung gemäß der Erfindung kombiniert werden können. Es können auch einzelne Teile der vorbeschriebenen Merkmalskombination für sich eigenständige, erfindungsgemäße Lösungen bilden.

### Bezugszeichenaufstellung

- 1: Injektionseinrichtung
- 2: Spritzenzylinder
- 3: Innenraum
- 4: Kolben
- 5: Kolbenstange
- 6: Injektionsnadel
- 7: Originalitätssicherungsvorrichtung
- 8: Hub
- 9: Anschlag
- 10: Innenfläche
- 11: Höhe
- 12: Kolbenring
- 13: Kolbenkörper
- 14: Steg
- 15: Steg
- 16: Schwächungsstelle
- 17: Nadelansatz
- 18: Gehäuse
- 19: Ansatz
- 20: Scheibe
- 21: Rückhalteglied
- 22: Umschaltorgan
- 23: Führungselement
- 24: Kunststoff
- 25: Kunststoff
- 26: Stützring
- 27: Durchgangsbohrung
- 28: Längsachse
- 29: Innenfläche
- 30: Öffnungswinkel
- 31: Öffnungswinkel
- 32: Außenfläche
- 33: Öffnung
- 34: Durchmesser
- 35: Durchmesser
- 36: Freiraum
- 37: Einlaßbereich
- 38: Weite
- 39: Begrenzungswand
- 40: Außendurchmesser
- 41: Innendurchmesser
- 42: Anschlagteil
- 43: Innenfläche
- 44: Bund
- 45:
- 46: Ausnehmung
- 47: Stirnseite
- 48: Dichtabschnitt
- 49: Führungsbahn
- 50: Führungszapfen
- 51: Führungssteg
- 52: Kanal
- 53: Medium
- 54: Führungslänge
- 55: Führungsnase
- 56: Drehkegelstumpf
- 57: Oberfläche
- 58: Durchmesser
- 59: Außendurchmesser
- 60: Entlüftungskanal
- 61: Stirnfläche
- 62: Aufnahmekammer
- 63: Leitkanal
- 64: O-Ring
- 65: Pfeil
- 66: Pfeil
- 67: Pfeil
- 68: Stirnseite
- 69: Pfeil
- 70: Öffnungswinkel
- 71: Länge
- 72: Gesamtlänge
- 73: Pfeil
- 74: Außendurchmesser
- 75: Stirnfläche
- 76: Seitenkante
- 77: Gerade
- 78: Winkel
- 79: Dichtansatz
- 80: Außendurchmesser
- 81: Außenumfangsfläche
- 82: Durchlaßkanal
- 83: Führungsbohrung
- 84: Kanal
- 85: Länge
- 86: Stirnkante
- 87: Halterippe
- 88: Steg
- 89: Durchmesser
- 90: Durchtrittskanal
- 91: Kernausnehmung
- 92: Ampulle
- 93: Luftblase
- 94: Körper
- 95: Gehäuseteil
- 96: Gehäuseteil
- 97: Stirnwand
- 98: Führungslänge
- 99: Distanz
- 100: Rückstellvorrichtung
- 101: Durchtrittsöffnung
- 102: Rückstellarm
- 103: Erhöhung
- 104: Pfeil
- 105: Pfeil
- 106: Pfeil
- 107: Hub
- 108: Nadellänge
- 109: Innendurchmesser
- 110: Entlüftungskanal
- 111: Führungselement
- 112: Distanz
- 113: Abstand
- 114: Distanz
- 115:
- 116:
- 117:
- 118:
- 119:
- 120:
- 121:
- 122:
- 123:
- 124:
- 125:
- 126:
- 127:
- 128:
- 129:
- 130:
- 131:
- 132:
- 133:
- 134:
- 135:
- 136:
- 137:
- 138:
- 139:
- 140:
- 141:
- 142:
- 143:
- 144:
- 145:
- 146:
- 147:
- 148:
- 149:
- 150:
- 151:
- 152:
- 153:
- 154:
- 155:
- 156:
- 157:
- 158:
- 158:
- 160:

## Patentansprüche

1. Injektionseinrichtung für Einmalverwendung mit einem Spritzenzylinder zur Aufnahme eines flüssigen Mediums, einer Injektionsnadel, einen im Innenraum des Spritzenzylinders verschiebbar gelagerten Kolben, der mit einer sich von diesem in einer der Injektionsnadel entgegengesetzten Richtung erstreckenden Kolbenstange bewegungsverbunden ist und mit einer durch das mit dem Kolben bewegte Medium betätigten Originalitätssicherungsvorrichtung, die in einem der Injektionsnadel zugewandten Einlaßbereich des Spritzenzylinders angeordnet ist und ein Umschaltorgan aufweist, welches einen Dichtabschnitt mit einer größeren Querschnittsfläche aufweist, als ein Durchtrittsquerschnitt einer Öffnung in einem Rückhalteglied und dessen Dichtabschnitt mit dem in radialer Richtung bzw. in Richtung der Injektionsnadel elastisch verformbaren kreisringförmigen Rückhalteglied auf der dem Kolben zugewandten Seite desselben in einer Ruhestellung gehalten ist und daß sich in einer Sperrstellung des Umschaltorgans der Dichtabschnitt auf der der Injektionsnadel zugewandten Seite des Rückhaltegliedes, das für das Umschaltorgan in Richtung des Kolbens als dichtender Anschlag ausgebildet ist befindet und daß zwischen dem Rückhalteglied und der Injektionsnadel ein Aufnahmeraum für das Umschaltorgan bzw. dessen Dichtabschnitt angeordnet ist, dadurch gekennzeichnet, daß das Rückhalteglied (21) aus einem elastisch rückstellbaren Material hergestellt und in einem in den Innenraum (3) des Spritzenzylinders (2) eingesetzten Gehäuse (18) der Originalitätssicherungsvorrichtung (7) aus einem gegenüber dem Material des Rückhaltegliedes (21) härteren Material positioniert gehalten oder an dieses angeformt ist und daß das Umschaltorgan (22) ein an den Dichtabschnitt (48) anschließendes Führungselement (23) aufweist, welches in einer Führungsbahn (49) des Gehäuses (18) bzw. im Spritzenzylinder (2) geführt ist und daß eine Führungslänge zwischen dem Führungselement (23) und der Führungsbahn (49) länger ist, als zumindest eine Distanz zwischen der Ruhe- und Sperrstellung des Umschaltorgans (22).

2. Injektionseinrichtung nach Anspruch 1, dadurch gekennzeichnet, daß das Führungselement (23) durch einen sich in Richtung der Längsachse (28) der Injektionseinrichtung (1) erstreckenden Führungszapfen (50) gebildet ist und eine Gesamtlänge (72) des Umschaltorgans (22) größer ist als das Zweifache einer Länge (71) des Dichtabschnittes (48).

3. Injektionseinrichtung nach Anspruch 1 oder 2, dadurch gekennzeichnet, daß eine Länge des Führungszapfens (50) ein Mehrfaches der Länge (71) des Dichtabschnittes (48) entspricht.

4. Injektionseinrichtung nach einem der Ansprüche 1 bis 3, dadurch gekennzeichnet, daß der Führungszapfen (50) in einer die Führungsbahn (49) bildenden das Gehäuse (18) in Richtung der Längsachse (28) der Injektionseinrichtung (1) durchsetzenden Führungsbohrung (83) annähernd spielfrei, d.h. mit geringer Toleranz in der Führungsbohrung (83) längsverschieblich geführt ist.

5. Injektionseinrichtung nach einem der Ansprüche 1 bis 4, dadurch gekennzeichnet, daß in der Führungsbohrung (83) parallel zur Längsachse (28) der Injektionseinrichtung (1) verlaufende über den Umfang derselben verteilt und distanziert voneinander angeordnete, durch nutförmige Vertiefungen gebildete Kanäle (84) angeordnet sind.

6. Injektionseinrichtung nach einem der Ansprüche 1 bis 5, dadurch gekennzeichnet, daß das Gehäuse (18) in seinem der Injektionsnadel (6) zugewandten Stirnendbereich mit einer zylindrischen Ausnehmung (46) zur Aufnahme des durch eine kreisringförmige Scheibe gebildeten Rückhaltegliedes versehen ist und eine Stirnseite (47) des Gehäuses (18) sowie die Scheibe an einem Anschlag im Spritzenzylinder (2) anliegt.

7. Injektionseinrichtung nach einem der Ansprüche 1 bis 6, dadurch gekennzeichnet, daß sich die Führungsbohrung (83) und/oder die Kanäle (84) von dieser Ausnehmung (46) in Richtung der Längsachse (28) der Injektionseinrichtung (1) bis in den Bereich einer gegenüberliegenden dem Kolben (4) zugewandten Stirnwand erstreckt bzw. erstrecken.

8. Injektionseinrichtung nach einem der Ansprüche 1 bis 7, dadurch gekennzeichnet, daß ein Durchmesser der Ausnehmung (46) einem Außendurchmesser (40) des Rückhaltegliedes (21) entspricht und größer ist als ein Innendurchmesser (41) eines kreisringförmigen Ansatzes (19) für das Gehäuse (18) im Spritzenzylinder (2).

9. Injektionseinrichtung nach einem der Ansprüche 1 bis 8, dadurch gekennzeichnet, daß das Gehäuse (18) aus mehreren Gehäuseteilen (95, 96) besteht und das Rückhalteglied (21) zwischen den beiden Gehäuseteilen (95, 96) eingesetzt und positioniert gehalten ist.

10. Injektionseinrichtung nach einem der Ansprüche 1 bis 9, dadurch gekennzeichnet, daß die Gehäuseteile (95, 96) miteinander verklebt oder über eine Schweißverbindung, insbesondere eine Ultraschallschweißung oder eine Schnapp- bzw. Rastverbindung miteinander verbunden sind.

11. Injektionseinrichtung nach einem der Ansprüche 1 bis 10, dadurch gekennzeichnet, daß der Dichtabschnitt (48) durch einen an den Führungszapfen (50) anschließenden Kugelkalottenabschnitt gebildet ist, dessen Außendurchmesser (59) größer ist als ein Außendurchmesser (74) des Führungszapfens (50) und der mit zunehmendem Abstand vom Führungszapfen (50) um eine zunehmende Höhe über dessen Umfang vorragt und daß ein dem Führungszapfen (50) gegenüberliegender Endteil des Umschaltorgans (22) durch einen Drehkegelstumpf gebildet ist.

12. Injektionseinrichtung nach einem der Ansprüche 1 bis 11, dadurch gekennzeichnet, daß ein Öffnungswinkel (70) des Drehkegelstumpfes (56) 90° oder kleiner ist.

13. Injektionseinrichtung nach einem der Ansprüche 1 bis 12, dadurch gekennzeichnet, daß der Dichtabschnitt (48) durch eine Kugelkalotte gebildet ist, deren Außendurchmesser (59) größer ist als der Außendurchmesser (74) des Führungszapfens (50) und deren kreisförmige Stirnfläche (61) einen Durchmesser (58) aufweist, der kleiner ist als der Außendurchmesser (74) des Führungszapfens (50) und der Durchmesser (34) der Öffnung (33) des Rückhaltegliedes (21) bzw. des Stützringes (26).

14. Injektionseinrichtung nach einem der Ansprüche 1 bis 13, dadurch gekennzeichnet, daß der Durchmesser (34) der Öffnung (33) des Stützringes (26) kleiner ist als der Außendurchmesser (74) des Führungszapfens (50).

15. Injektionseinrichtung nach einem der Ansprüche 1 bis 14, dadurch gekennzeichnet, daß der Stützring (26) als Drehkegelmantelabschnitt gebildet ist, der einen Durchmesser (35) eines Basiskreises aufweist, der größer ist als ein maximaler Außendurchmesser (59) des Dichtabschnittes (48) und dessen Durchmesser (34) der Öffnung (33) kleiner ist als ein Außendurchmesser (59) des Dichtabschnittes (48) und bevorzugt kleiner als ein Außendurchmesser (74) des Führungszapfens (50).

16. Injektionseinrichtung nach einem der Ansprüche 1 bis 15, dadurch gekennzeichnet, daß ein Öffnungswinkel (30) der dem Dichtabschnitt (48) in der Ruhestellung zugewandten Innenflächen (29) des Stützringes (26) 90° oder größer ist.

17. Injektionseinrichtung nach einem der Ansprüche 1 bis 16, dadurch gekennzeichnet, daß der Stützring (26) sowohl in Längsrichtung der Injektionseinrichtung als auch in radialer Richtung gegenüber dem Spritzenzylinder (2) freigestellt ist.

18. Injektionseinrichtung nach einem der Ansprüche 1 bis 17, dadurch gekennzeichnet, daß das Rückhalteglied (21) bzw. der dessen Öffnung (33) umgebender Stützring (26) aus einem Elastomer, z.B. einem Pharmagummi, Silikonkautschuk oder dgl., insbesondere mit einer Härte von Shore A 40 bis 65 hergestellt ist.

19. Injektionseinrichtung nach einem der Ansprüche 1 bis 18, dadurch gekennzeichnet, daß der Stützring (26) aus elastisch dehnbarem und rückstellbaren Material, insbesondere Kunststoff (25), Pharmagummi, Silikon oder dgl. besteht.

20. Injektionseinrichtung nach einem der Ansprüche 1 bis 19, dadurch gekennzeichnet, daß eine Stirnfläche (75) des Stützrings (26) senkrecht bzw. tangential zu der dem Führungszapfen (50) zugeordneten Oberfläche (57) des Dichtabschnittes (48) ausgerichtet ist.

21. Injektionseinrichtung nach einem der Ansprüche 1 bis 20, dadurch gekennzeichnet, daß der Spritzenzylinder (2) insbesondere im Bereich eines die Injektionsnadel (6) aufnehmenden Halteansatz im wesentlichen an die Außenform des Umschaltorgans (22) bzw. des Dichtabschnittes (48) angepaßt ist.

22. Injektionseinrichtung nach einem der Ansprüche 1 bis 21, dadurch gekennzeichnet, daß zwischen dem Rückhalteglied (21) und der Injektionsnadel (6) eine in Richtung des Kolbens (4) wirksame Rückstellvorrichtung (100) angeordnet ist und ein Abstand zwischen den Rückstellarmen (102) der Rückstellvorrichtung (100) und der Stirnfläche (61) des Rückhaltegliedes (21) parallel zur Längsachse (28) bzw. der Führungsbahn (49) gleich oder kleiner ist als eine Länge (71) des Dichtabschnittes (48).

23. Injektionseinrichtung nach einem der Ansprüche 1 bis 22, dadurch gekennzeichnet, daß eine Rückhaltekraft des Rückhaltegliedes (21) in Richtung der Injektionsnadel (6) kleiner ist, als eine auf den Kolben (4) einwirkende Vortriebskraft und bevorzugt kleiner als 7,5 bis 10 N ist.

24. Injektionseinrichtung nach einem der Ansprüche 1 bis 23, dadurch gekennzeichnet, daß die Rückstellarme (102) der Rückstellvorrichtung (100) als Federarme ausgebildet und elastisch verformbar sind.

25. Injektionseinrichtung nach einem der Ansprüche 1 bis 24, dadurch gekennzeichnet, daß das Gehäuse (18) bzw. das Rückhalteglied (21) in Richtung der Längsachse (28) der Injektionseinrichtung zwischen einer in der der Injektionsnadel (6) zugewandten Stirnwand (97) des Innenraums (3) angeordneten Anschlag und einen in den Innenraum (3) vorragenden Anschlagteil (42), beispielsweise eine Noppe oder einen Wulst gehalten ist.

26. Injektionseinrichtung nach einem der Ansprüche 1 bis 25, dadurch gekennzeichnet, daß eine Längsdistanz zwischen der Stirnwand (97) und den Anschlagteilen (42) größer ist als eine zwischen diesen befindliche Baulänge des Gehäuses (18).

27. Injektionseinrichtung nach einem der Ansprüche 1 bis 26, dadurch gekennzeichnet, daß das Produkt aus der Differenz zwischen der Längsdistanz und der Baulänge des Gehäuses (18) und der senkrecht zur Längsachse (28) angeordneten Querschnittsfläche um 5 bis 30 %, bevorzugt 10 % größer ist, als das Volumen der Injektionsnadel (6) und eines gegebenenfalls zwischen dieser und dem Innenraum (3) des Spritzenzylinders (2) angeordneten Nadelkanals.

28. Injektionseinrichtung nach einem der Ansprüche 1 bis 27, dadurch gekennzeichnet, daß eine Distanz (112) zwischen der Stirnkante (86) des Rückhaltegliedes (21) in bei maximaler Rückstellkraft verformter Stellung und den Rückstellarmen (102) größer ist, als ein Abstand (113) zwischen den am Rückhalteglied (21) und den an den Rückstellarmen (102) anliegenden Wandbereichen des Umschaltorgans (22).

29. Injektionseinrichtung nach einem der Ansprüche 1 bis 28, dadurch gekennzeichnet, daß ein sich aus einem Produkt aus einer Differenz zwischen einer Distanz (99) sowie der Führungslänge (98) und der Querschnittsfläche einer Stirnseite (68) des Führungsansatzes ergebende Volumen gleich oder größer ist dem Volumen des Kanals der Injektionsnadel (6) und eines eventuell zwischen dieser und dem Innenraum (3) des Spritzenzylinders (2) angeordneten Nadelkanals.

30. Injektionseinrichtung nach einem der Ansprüche 1 bis 29, dadurch gekennzeichnet, daß die Führungsbahn (49) für den Führungszapfen im Nadelansatz (17) des Spritzenzylinders (2) für die Injektionsnadel (6) angeordnet ist.

31. Injektionseinrichtung nach einem der Ansprüche 1 bis 30, dadurch gekennzeichnet, daß der Spritzenzylinder (2) in seinem einen Endbereich mit einer Stirnwand (97) verschlossen und sein gegenüberliegendes Ende offen ist, und daß vom offenen Ende her eine Kolbenstange (5) in den Innenraum (3) des Spritzenzylinders (2) ragt, mit der der Kolben (4), der mit der Kolbenstange (5) bewegungsverbunden ist, in den Innenraum (3) des Spritzenzylinders (2) hineingeschoben bzw. aus diesem herausgezogen werden kann, wobei die Stirnwand (97) mit einem Nadelkanal bzw. der Injektionsnadel (6) durchsetzt ist, und daß an der Stirnwand (97) ein in den Innenraum (3) des Spritzenzylinders (2) eingesetztes Gehäuse (18) anliegt, welches über Dichtmittel dichtend in den Innenraum (3) des Spritzenzylinders (2) eingesetzt ist und aus einem nadelseitigen, an der Stirnwand (97) anliegenden und einem kolbenseitigen Gehäuseteil (95, 96) besteht, die sich bevorzugt einander überlappen und eine in Längsrichtung durchgehende Öffnung aufweisen, und daß zwischen dem Kolben (4) und dem nadelseitigen Gehäuseteil (96) ein Rückhalteglied (21) für ein Umschaltorgan (22) angeordnet ist, welches einen zylindrischen Führungszapfen (50) aufweist, der in einer diesen umgebenden Bohrung in einem der beiden Gehäuseteile (95, 96) oder im Spritzenzylinder (2) mit geringem Spiel geführt ist und daß das Umschaltorgan (22) in Auspreßrichtung des Mediums (53) mit einem stirnseitig sich verjüngenden Ende versehen ist und zwischen diesem Ende und dem Führungszapfen (50) ein Dichtabschnitt (48) ausgebildet ist, welcher eine größere Querschnittsfläche als die Öffnung (33) in einem Rückhalteglied (21) aufweist, wobei beim Einwirken eines unter Druck stehenden Mediums (53) auf die Innenstirnseite des Führungszapfens (50) das Umschaltorgan (22) durch das Rückhalteglied (21) hindurch in die zwischen dem Rückhalteglied (21) und den Nadelkanal liegende Aufnahmekammer (62) gedrückt wird und sich der Stützring (26) des Rückhaltegliedes (21) gegen den Dichtabschnitt (48) abstützt und eine Rückwärtsbewegung des Umschaltorgans (22) in Richtung des Kolbens (4) und einen Durchtritt des Mediums (53) zwischen dem Umschaltorgan (22) und dem Rückhalteglied (21) auch bei einer Druckbelastung durch den Nadelkanal verhindert, wobei der Führungszapfen (50) noch immer in der Führungsbahn (49) geführt ist.

32. Injektionseinrichtung nach einem der Ansprüche 1 bis 31, dadurch gekennzeichnet, daß die Längsachse des Umschaltorgans (22) koaxial zur Längsachse (28) des Nadelkanals angeordnet ist.

33. Injektionseinrichtung nach einem der Ansprüche 1 bis 32, dadurch gekennzeichnet, daß die Längsachse des Umschaltorgans (22) exzentrisch zur Längsachse (28) des Nadelkanals angeordnet ist.

34. Injektionseinrichtung nach einem der Ansprüche 1 bis 33, dadurch gekennzeichnet, daß das Umschaltorgan (22) in seinem in der Ruhestellung am Rückhalteglied (21) bzw. dem Stützring anliegenden Oberflächenbereich einen in Längsrichtung des Umschaltorgans (22) verlaufenden Durchtrittskanal (90) aufweist, dessen Durchtrittsquerschnitt kleiner ist als ein Mindestquerschnitt zum Durchtritt einer Flüssigkeit mit einer vorbestimmbaren Viskosität.

35. Injektionseinrichtung nach einem der Ansprüche 1 bis 34, dadurch gekennzeichnet, daß auf einer dem Umschaltorgan (22) zugewandten Innenfläche (29) des Stützringes (26) eine in etwa in Längsrichtung des Spritzenzylinders (2) verlaufende Vertiefung angeordnet ist.

36. Injektionseinrichtung nach einem der Ansprüche 1 bis 35, dadurch gekennzeichnet, daß auf der dem Rückhalteglied (21) zugewandten Oberfläche (56) des Umschaltorgans (22) bzw. der dem Umschaltorgan (22) zugewandten Innenfläche (29) des Rückhaltegliedes (21) zumindest ein eine Erhöhung (103) bildender vorragender Teil, insbesondere ein in Längsrichtung des Spritzenzylinders (2) verlaufender Steg angeordnet ist.

## Claims

1. Single use injection device having an injection cylinder for receiving a liquid medium, an injection needle, a piston mounted displaceably in the interior of the injection cylinder and which is movably connected to a piston rod extending from the piston in a direction opposite to the injection nozzle and having an authenticity security device actuated by the medium moved by the piston and which is arranged in an inlet region of the injection cylinder facing the injection needle, and has a switching member, which has a sealing section having a greater cross-sectional area than a passage cross-section of an opening in a retaining element and the sealing section thereof is held in the rest position by the circular retaining element which is resiliently deformable in the radial direction or in the direction of the injection needle on the side of the same facing the piston, and that in a locking position for the switching member the sealing section is situated on the side of the retaining element facing the injection needle and is designed as a sealing stop for the switching member in the direction of the piston, and that a receiving space for the switching member or the sealing section thereof is arranged between the retaining element and the injection needle, characterised in that the retaining element (21) is produced from a resiliently restorable material and is held in position in a housing (18) of the authenticity security device (7), made from a material which is harder compared to the material of the retaining element (21), and inserted in the interior (3) of the injection cylinder (2), or moulded onto it, and in that the switching member (22) has a guide element (23) connected to the sealing section (48) which is guided in a guide track (49) of the housing (18) or in the injection cylinder (2), and in that a guide length between the guide element (23) and the guide track (49) is longer than at least a distance between the rest position and the locking position of the switching member (22).

2. Injection device according to claim 1, characterised in that the guide element (23) is formed by a guide pin (50) extending in the direction of the longitudinal axis (28) of the injection device (1), and a total length (72) of the switching member (22) is greater than twice a length (71) of the sealing section (48).

3. Injection device according to claim 1 or 2, characterised in that a length of the guide pin (50) corresponds to a multiple of the length (71) of the sealing section (48).

4. Injection device according to one of claims 1 to 3, characterised in that the guide pin (50) is guided to be longitudinally displaceable in a guide bore (83) forming the guide track (49) and penetrating the housing (18) in the direction of the longitudinal axis (28) of the injection device (1), approximately without clearance, that is with low tolerance in the guide bore (83).

5. Injection device according to one of claims 1 to 4, characterised in that channels (84) extending parallel to the longitudinal axis (28) of the injection device (1), distributed over the periphery of the same and arranged at a distance from one another, and formed by groove-like depressions, are arranged in the guide bore (83).

6. Injection device according to one of claims 1 to 5, characterised in that the end-face region facing the injection needle (6) of the housing (18) is provided with a cylindrical recess (46) for receiving the retaining element formed by a circular disc, and an end-face side (47) of the housing (18) and the disc rests against a stop in the injection cylinder (2).

7. Injection device according to one of claims 1 to 6, characterised in that the guide bore (83) and/or the channels (84) extends or extend from this recess (46) in the direction of the longitudinal axis (28) of the injection device (1) as far as the region of an opposite end-face wall facing the piston (4).

8. Injection device according to one of claims 1 to 7, characterised in that a diameter of the recess (46) corresponds to an external diameter (40) of the retaining element (21) and is greater than an internal diameter (41) of a circular attachment (19) for the housing (18) in the injection cylinder (2).

9. Injection device according to one of claims 1 to 8, characterised in that the housing (18) comprises several housing parts (95, 96) and the retaining element (21) is inserted between the two housing parts (95, 96) and held in position.

10. Injection device according to one of claims 1 to 9, characterised in that the housing parts (95, 96) are adhered to one another or connected to one another via a weld connection, in particular an ultrasound weld, or a snap or lock connection.

11. Injection device according to one of claims 1 to 10, characterised in that the sealing section (48) is formed by a calotte shell section connected to the guide pin (50), the external diameter (59) of which is greater than an external diameter (74) of the guide pin (50) and which with increasing distance from the guide pin (50) projects by an increasing height over its periphery, and in that an end part of the switching member (22) opposite the guide pin (50) is formed by a frustum.

12. Injection device according to one of claims 1 to 11, characterised in that an opening angle (70) for the frustum (56) is 90° or smaller.

13. Injection device according to one of claims 1 to 12, characterised in that the sealing section (48) is formed by a calotte shell, the external diameter (59) of which is greater than the external diameter (74) of the guide pin (50), and the circular end-face (61) thereof has a diameter (58) which is smaller than the external diameter (74) of the guide pin (50), and the diameter (34) of the opening (33) of the retaining element (21) or the supporting ring (26).

14. Injection device according to one of claims 1 to 13, characterised in that the diameter (34) of the opening (33) of the supporting ring (26) is smaller than the external diameter (74) of the guide pin (50).

15. Injection device according to one of claims 1 to 14, characterised in that the supporting ring (26) is formed as a cone envelope section, which has a diameter (35) of a base circle which is greater than a maximum external diameter (59) of the sealing section (48), and its diameter (34) of the opening (33) is smaller than an external diameter (59) of the sealing section (48) and preferably smaller than an external diameter (74) of the guide pin (50).

16. Injection device according to one of claims 1 to 15, characterised in that an opening angle (30) of the inner surfaces (29) of the supporting ring (26) facing the sealing section (48) in the rest position, is 90° or greater.

17. Injection device according to one of claims 1 to 16, characterised in that the supporting ring (26) is released with respect to the injection cylinder (2) both in the longitudinal direction of the injection device and in the radial direction.

18. Injection device according to one of claims 1 to 17, characterised in that the retaining device (21) or the supporting ring (26) surrounding the opening (33) thereof is made from an elastomer, for example a pharmacological rubber, silicone rubber or the like, in particular having a Shore A hardness of 40 to 65.

19. Injection device according to one of claims 1 to 18, characterised in that the supporting ring (26) consists of a resiliently expandable and restorable material, in particular plastic (25), pharmacological rubber, silicone or the like.

20. Injection device according to one of claims 1 to 19, characterised in that an end-face (75) of the supporting ring (26) is aligned vertically or tangentially to the surface (57) of the sealing section (48) assigned to the guide pin (50).

21. Injection device according to one of claims 1 to 20, characterised in that the injection cylinder (2), particularly in the region of a retaining attachment receiving the injection needle (6), is essentially adapted to the external shape of the switching member (22) or the sealing section (48).

22. Injection device according to one of claims 1 to 21, characterised in that a resetting device (100) effective in the direction of the piston (4) is arranged between the retaining element (21) and the injection needle (6), and a distance between the resetting arms (102) of the resetting device (100) and the end-face (61) of the retaining element (21) parallel to the longitudinal axis (28) or the guide track (49), is equal to or smaller than a length (71) of the sealing section (48).

23. Injection device according to one of claims 1 to 22, characterised in that a retaining force of the retaining element (21) in the direction of the injection needle (6) is smaller than a propulsive force acting on the piston (4), and is preferably less than 7.5 to 10 N.

24. Injection device according to one of claims 1 to 23, characterised in that the resetting arms (102) of the resetting device (100) are designed as spring arms and can be resiliently deformed.

25. Injection device according to one of claims 1 to 24, characterised in that the housing (18) or the retaining element (21) is held in the direction of the longitudinal axis (28) of the injection device between a stop arranged in the end-face wall (97) of the interior (3) facing the injection needle (6) and a stop part (42) projecting into the interior (3), for example a knob or a bead.

26. Injection device according to one of claims 1 to 25, characterised in that a longitudinal distance between the end-face wall (97) and the stop parts (42) is greater than an overall length of the housing (18) situated between them.

27. Injection device according to one of claims 1 to 26, characterised in that the product of the difference between the longitudinal distance and the overall length of the housing (18) and the cross-sectional area arranged vertically to the longitudinal axis (28), is greater by 5 to 30 %, preferably 10 %, than the volume of the injection needle (6) and a needle channel arranged optionally between the injection needle (6) and the interior (3) of the injection cylinder (2).

28. Injection device according to one of claims 1 to 27, characterised in that a distance (112) between the end-face edge (86) of the retaining element (21) in deformed position at maximum restoring force and the resetting arms (102), is greater than a distance (113) between the wall regions of the switching member (22) resting against the retaining element (21) and those resting against the resetting arms (102).

29. Injection device according to one of claims 1 to 28, characterised in that a volume resulting from a product of the difference between a distance (99) and the guide length (98) and the cross-sectional area of an end-face side (68) of the guide attachment, is the same or greater than the volume of the channel of the injection needle (6) and a needle channel possibly arranged between the injection needle (6) and the interior (3) of the injection cylinder (2).

30. Injection device according to one of claims 1 to 29, characterised in that the guide track (49) for the guide pins is arranged in the needle attachment (17) of the injection cylinder (2) for the injection needle (6).

31. Injection device according to one of claims 1 to 30, characterised in that one end region of the injection cylinder (2) is sealed by an end-face wall (97) and its opposite end is open, and in that, starting from the open end, a piston rod (5) projects into the interior (3) of the injection cylinder (2), by means of which the piston (4), which is movably connected to the piston rod (5), may be pushed into the interior (3) of the injection cylinder (2) or withdrawn from it, wherein the end-face wall (97) is penetrated by a needle channel or the injection needle (6), and in that a housing (18) inserted in the interior (3) of the injection cylinder (2) rests against the end-face wall (97) and it is inserted in sealing manner in the interior (3) of the injection cylinder (2) by means of sealing agent and comprises a needle-side housing part (96) resting against the end-face wall (97) and a piston-side housing part (95) which preferably overlap one another and have an opening passing through in the longitudinal direction, and in that a retaining element (21) for a switching member (22) is arranged between the piston (4) and the needle-side housing part (96), the switching member (22) has a cylindrical guide pin (50) which is guided with low clearance in a bore surrounding it in one of the two housing parts (95, 96) or in the injection cylinder (2), and in that the switching member (22) in the expressing direction of the medium (53) is provided with an end tapering at the end-face side, and a sealing section (48) is formed between this end and the guide pin (50) and has a greater cross-sectional area than the opening (33) in a retaining element (21), wherein when a medium (53) under pressure acts on the inner end-face side of the guide pin (50), the switching member (22) is pressed through the retaining element (21) into the receiving chamber (62) lying between the retaining element (21) and the needle channel, and the supporting ring (26) of the retaining element (21) is supported against the sealing section (48) and prevents rearward movement of the switching member (22) in the direction of the piston (4) and the passage of medium (53) between the switching member (22) and the retaining element (21) even when there is pressure load through the needle channel, wherein the guide pin (50) is still guided in the guide track (49).

32. Injection device according to one of claims 1 to 31, characterised in that the longitudinal axis of the switching member (22) is arranged coaxially to the longitudinal axis (28) of the needle channel.

33. Injection device according to one of claims 1 to 32, characterised in that the longitudinal axis of the switching member (22) is arranged eccentrically to the longitudinal axis (28) of the needle channel.

34. Injection device according to one of claims 1 to 33, characterised in that the surface region of the switching member (22) resting against the retaining element (21) or the supporting ring in the rest position, has a passage channel (90) extending in the longitudinal direction of the switching member (22), the passage cross-section of which is smaller than a minimum cross-section for passage of a liquid having a pre-determinable viscosity.

35. Injection device according to one of claims 1 to 34, characterised in that a depression extending approximately in the longitudinal direction of the injection cylinder (2) is arranged on an inner surface (29) of the supporting ring (26) facing the switching member (22).

36. Injection device according to one of claims 1 to 35, characterised in that at least one projecting part forming a raised section (103), in particular a web extending in the longitudinal direction of the injection cylinder (2), is arranged on the surface (56) of the switching member (22) facing the retaining element (21) or the inner surface (29) of the retaining element (21) facing the switching member (22).

## Revendications

1. Dispositif d'injection à usage unique comprenant un cylindre d'injection servant de logement à un milieu liquide, une aiguille d'injection, un piston monté en coulissement dans l'espace interne du cylindre d'injection, le piston étant relié en mobilité avec une tige de piston s'étendant à partir de ce dernier dans une direction opposée à celle de l'aiguille d'injection, ainsi qu'un dispositif de sécurité original actionné par le milieu mis en mouvement par le piston, qui est disposé dans une zone d'entrée du cylindre d'injection tournée vers l'aiguille d'injection et qui présente un organe de commutation qui présente une section d'étanchéification dont l'aire de section est supérieure à la section de passage d'une ouverture pratiquée dans un élément de retenue et dont la section d'étanchéification est maintenue, dans la position de repos, à l'aide de l'élément de retenue en forme d'anneau de cercle élastiquement déformable en direction radiale, respectivement dans la direction de l'aiguille d'injection, contre le côté de l'élément de retenue tourné vers le piston, la section d'étanchéification, dans la position de verrouillage de l'organe de commutation, se trouvant sur le côté de l'élément de retenue tourné vers l'aiguille d'injection, qui est réalisé sous forme de butée d'étanchéification pour l'organe de commutation dans la direction du piston, et un espace de réception pour l'organe de commutation, respectivement pour sa section d'étanchéification étant ménagé entre l'élément de retenue et l'aiguille d'injection, caractérisé en ce que l'élément de retenue (21) est fabriqué en une matière élastiquement résiliente et est maintenu en position dans ou façonné contre un logement (18) du dispositif de sécurité original (7), inséré dans l'espace interne (3) du cylindre d'injection (2), le dispositif de sécurité étant réalisé en une matière plus dure que la matière de l'élément de retenue (21), et en ce que l'organe de commutation (22) présente un élément de guidage (23) limitrophe à la section d'étanchéification (48), qui est guidé dans une voie de guidage (49) du logement (18), respectivement dans le cylindre d'injection (2), et en ce que la longueur minimale de guidage entre l'élément de guidage (23) et la voie de guidage (49) est supérieure à la distance entre la position de repos et la position de verrouillage de l'organe de commutation (22).

2. Dispositif d'injection selon la revendication 1, caractérisé en ce que l'élément de guidage (23) est formé par un tourillon de guidage (50) s'étendant dans la direction de l'axe longitudinal (28) du dispositif d'injection (1), et la longueur totale (72) de l'organe de commutation (22) est supérieure au double de la longueur (71) de la section d'étanchéification (48).

3. Dispositif d'injection selon la revendication 1 ou 2, caractérisé en ce que la longueur du tourillon de guidage (50) représente un multiple de la longueur (71) de la section d'étanchéification (48).

4. Dispositif d'injection selon l'une quelconque des revendications 1 à 3, caractérisé en ce que le tourillon de guidage (50) est guidé dans un alésage de guidage (83) formant la voie de guidage (49) et traversant le logement (18) dans la direction de l'axe longitudinal (28) du dispositif d'injection (1), approximativement sans jeu, c'est-à-dire est guidé en coulissement longitudinal avec une tolérance minime dans l'alésage de guidage (83).

5. Dispositif d'injection selon l'une quelconque des revendications 1 à 4, caractérisé en ce que, dans l'alésage (83), sont disposés des canaux (84) formés par des renfoncements en forme de rainures, s'étendant parallèlement à l'axe longitudinal (28) du dispositif d'injection (1), en étant disposés de telle sorte qu'ils sont répartis sur la périphérie de ce dernier et à distance l'un de l'autre.

6. Dispositif d'injection selon l'une quelconque des revendications 1 à 5, caractérisé en ce que le logement (18) est muni, dans sa zone frontale tournée vers l'aiguille d'injection (6), d'un évidement cylindrique (46) pour que vienne s'y loger l'élément de retenue formé par un disque en forme d'anneau de cercle, le côté frontal (47) du logement (18), ainsi que le disque venant s'appuyer contre une butée prévue dans le cylindre d'injection (2).

7. Dispositif d'injection selon l'une quelconque des revendications 1 à 6, caractérisé en ce que l'alésage de guidage (83) et/ou les canaux (84) s'étend, respectivement s'étendent depuis cet évidement (46) en direction de l'axe longitudinal (28) du dispositif d'injection (1) jusque dans la zone de la paroi frontale opposée tournée vers le piston (4).

8. Dispositif d'injection selon l'une quelconque des revendications 1 à 7, caractérisé en ce que le diamètre de l'évidement (46) correspond au diamètre externe (40) de l'élément de retenue (21) et est supérieur au diamètre interne (41) de la pièce rapportée (19) en forme d'anneau de cercle destiné au logement (18) dans le cylindre d'injection (2).

9. Dispositif d'injection selon l'une quelconque des revendications 1 à 8, caractérisé en ce que le logement (18) est constitué par plusieurs parties de logement (95, 96) et l'élément de retenue (21) est inséré et maintenu en position entre les deux parties de logement (95, 96).

10. Dispositif d'injection selon l'une quelconque des revendications 1 à 9, caractérisé en ce que les parties de logement (95, 96) sont collées l'une à l'autre ou bien sont reliées l'une à l'autre via une liaison par soudage, en particulier par soudage par ultrasons ou encore via une liaison par déclic, respectivement par encliquetage.

11. Dispositif d'injection selon l'une quelconque des revendications 1 à 10, caractérisé en ce que la section d'étanchéification (48) est formée par une section de calotte sphérique limitrophe au tourillon de guidage (50), dont le diamètre externe (59) est supérieur au diamètre externe (74) du tourillon de guidage (50) et fait saillie, lorsque sa distance par rapport au tourillon de guidage (50) augmente, d'une hauteur croissante, au-delà de la périphérie de ce dernier, et en ce que la forme de la partie terminale de l'organe de commutation (22) opposée au tourillon de guidage (50) correspond à celle d'un cône tronqué rotatif.

12. Dispositif d'injection selon l'une quelconque des revendications 1 à 11, caractérisé en ce que l'angle formé par l'ouverture (70) du cône tronqué rotatif (56) est égal ou inférieur à 90°.

13. Dispositif d'injection selon l'une quelconque des revendications 1 à 12, caractérisé en ce que la section d'étanchéification (48) est formée par une calotte sphérique dont le diamètre externe (59) est supérieur au diamètre externe (74) du tourillon de guidage (50) et dont la surface frontale circulaire (61) présente un diamètre qui est inférieur au diamètre externe (74) du tourillon de guidage (50) et au diamètre (34) de l'ouverture (33) de l'élément de retenue (21), respectivement de l'anneau de support (26).

14. Dispositif d'injection selon l'une quelconque des revendications 1 à 13, caractérisé en ce que le diamètre (34) de l'ouverture (33) de l'anneau de support (26) est inférieur au diamètre externe (74) du tourillon de guidage (50).

15. Dispositif d'injection selon l'une quelconque des revendications 1 à 14, caractérisé en ce que la forme de l'anneau de support (26) correspond à une section d'aire latérale de cône rotative qui présente un diamètre (35) d'un cercle de base qui est supérieur au diamètre externe maximal (59) de la section d'étanchéification (48), le diamètre (34) de l'ouverture (33) étant inférieur au diamètre externe (59) de la section d'étanchéification (48) et de préférence inférieur au diamètre externe (74) du tourillon de guidage (50).

16. Dispositif d'injection selon l'une quelconque des revendications 1 à 15, caractérisé en ce que l'angle d'ouverture (30) formé par les surfaces internes (29) de l'anneau de support (26) tournées vers la section d'étanchéification (48) dans la position de repos, est égal ou supérieur à 90°.

17. Dispositif d'injection selon l'une quelconque des revendications 1 à 16, caractérisé en ce que l'anneau de support (26) est disposé pour pouvoir se déplacer librement par rapport au cylindre d'injection (2) aussi bien dans la direction longitudinale du dispositif d'injection qu'en direction radiale.

18. Dispositif d'injection selon l'une quelconque des revendications 1 à 17, caractérisé en ce que l'élément de retenue (21), respectivement l'anneau de support (26) entourant son ouverture (33), est fabriqué à partir d'un élastomère, par exemple un caoutchouc à usage pharmaceutique, un caoutchouc de silicone ou analogues, présentant en particulier une dureté Shore A de 40 à 65.

19. Dispositif d'injection selon l'une quelconque des revendications 1 à 18, caractérisé en ce que l'anneau de support (26) est constitué par une matière manifestant une résilience et un allongement élastique, en particulier une matière synthétique (25), du caoutchouc à usage pharmaceutique, du silicone ou analogues.

20. Dispositif d'injection selon l'une quelconque des revendications 1 à 19, caractérisé en ce que la surface frontale (75) de l'anneau de support (26) est disposée en alignement perpendiculaire, respectivement tangentiel à la surface (57) de la section d'étanchéification (48) attribuée au tourillon de guidage (50).

21. Dispositif d'injection selon l'une quelconque des revendications 1 à 20, caractérisé en ce que le cylindre d'injection (2), en particulier dans la zone d'une pièce rapportée de retenue dans laquelle vient se loger l'aiguille d'injection (6), épouse essentiellement la forme externe de l'organe de commutation (22), respectivement de la section d'étanchéification (48).

22. Dispositif d'injection selon l'une quelconque des revendications 1 à 21, caractérisé en ce que, entre l'élément de retenue (21) et l'aiguille d'injection (6), est situé un dispositif de rappel (100) actif dans la direction du piston (4), la distance entre les bras de rappel (102) du dispositif de rappel (100) et la surface frontale (61) de l'élément de retenue (21) parallèlement à l'axe longitudinal (28), respectivement à la voie de guidage (49), est égale ou inférieure à la longueur (71) de la section d'étanchéification (48).

23. Dispositif d'injection selon l'une quelconque des revendications 1 à 22, caractérisé en ce que la force de retenue exercée par l'élément de retenue (21) dans la direction de l'aiguille d'injection (6) est inférieure à la force d'avancement s'exerçant sur le piston (4) et de préférence inférieure à 7,5-10 N.

24. Dispositif d'injection selon l'une quelconque des revendications 1 à 23, caractérisé en ce que les bras de rappel (102) du dispositif de rappel (100) sont réalisés en forme de bras de ressort et sont élastiquement déformables.

25. Dispositif d'injection selon l'une quelconque des revendications 1 à 24, caractérise en ce que le logement (18), respectivement l'élément de retenue (21) est maintenu, dans la direction de l'axe longitudinal (28) du dispositif d'injection, entre une butée disposée dans la paroi frontale (97) de l'espace interne (3), tournée vers l'aiguille d'injection (6), et un élément de butée (42) faisant saillie dans l'espace interne (3), par exemple un bouton ou une nervure.

26. Dispositif d'injection selon l'une quelconque des revendications 1 à 25, caractérisé en ce que la distance longitudinale entre la paroi frontale (97) et les éléments de butée (42) est supérieure à la longueur de montage du logement (18) s'étendant entre eux.

27. Dispositif d'injection selon l'une quelconque des revendications 1 à 26, caractérisé en ce que le produit de la différence entre la distance longitudinale et la longueur de montage du logement (18) et de l'aire de section disposée perpendiculairement à l'axe longitudinal (28) est supérieure de 5 à 30%, de préférence de 10% au volume de l'aiguille d'injection (6), et d'un canal d'aiguille éventuellement disposé entre cette dernière et l'espace interne (3) du cylindre d'injection (2).

28. Dispositif d'injection selon l'une quelconque des revendications 1 à 27, caractérisé en ce que la distance (112) entre l'arête frontale (86) de l'élément de retenue (21) dans la position déformée dans le cas d'une force de rappel maximale et les bras de rappel (102) est supérieure à la distance (113) entre les zones de parois de l'organe de commutation (22) adjacentes et celles adjacentes aux bras de rappel (102).

29. Dispositif d'injection selon l'une quelconque des revendications 1 à 28, caractérisé en ce que le volume que l'on obtient à partir du produit de la différence entre la distance (99), la longueur de guidage (98) et l'aire de section du côté frontal (68) de la pièce rapportée de guidage est égal ou supérieur au volume occupé par le canal de l'aiguille d'injection (6) et par le canal d'aiguille éventuellement disposé entre ce dernier et l'espace interne (3) du cylindre d'injection (2).

30. Dispositif d'injection selon l'une quelconque des revendications 1 à 29, caractérisé en ce que la voie de guidage (49) pour le tourillon de guidage est disposée dans la pièce rapportée d'aiguille (17) du cylindre d'injection (2) pour l'aiguille d'injection (6).

31. Dispositif d'injection selon l'une quelconque des revendications 1 à 30, caractérisé en ce que le cylindre d'injection (2) est fermé dans sa première zone terminale à l'aide d'une paroi frontale (97) et est ouvert à son extrémité opposée, et en ce qu'une tige de piston (5) fait saillie, à partir de l'extrémité ouverte, dans l'espace interne (3) du cylindre d'injection (2), la tige de piston (5), avec laquelle le piston (4) est relié en mobilité, permettant de pousser ce dernier dans l'espace interne (3) du cylindre d'injection (2), respectivement de l'en retirer, la paroi frontale (97) étant traversée par le canal d'aiguille, respectivement par l'aiguille d'injection (6), et en ce que, contre la paroi frontale (97), vient s'appuyer un logement (18) inséré dans l'espace interne (3) du cylindre d'injection (2), le logement étant inséré, via un joint d'étanchéification, de manière étanche dans l'espace interne (3) du cylindre d'injection (2) et étant constitué par la partie de logement (95) venant s'appuyer, côté aiguille, contre la paroi frontale (97) et par la partie de logement (96) côté piston, les deux parties se chevauchant de préférence et présentant une ouverture traversante en direction longitudinale, et en ce que, entre le piston (4) et la partie de logement (96) côté aiguille, est disposé un élément de retenue (21) pour un organe de commutation (22), qui présente un tourillon de guidage cylindrique (50) qui est guidé avec un jeu minime dans l'alésage entourant ce dernier pratiqué dans une des deux partics de logement (95, 96) ou dans le cylindre d'injection (2), et en ce que l'organe de commutation (22) est muni, dans la direction d'éjection du milieu (53), d'une extrémité se rétrécissant côté frontal, et une section d'étanchéification (48) est réalisée entre cette extrémité et le tourillon de guidage (50), qui présente une aire de section supérieure à l'ouverture (33) pratiquée dans l'élément de retenue (21), dans lequel, lorsqu'un milieu (53) se trouvant sous pression agit sur le côté frontal interne du tourillon de guidage (50), l'organe de commutation (22) est pressé par l'élément de retenue (21) à travers la chambre de réception (62) située entre l'élément de retenue (21) et le canal d'aiguille, et l'anneau de support (26) de l'élément de retenue (21) s'appuie contre la section d'étanchéification (48) et empêche un mouvement vers l'arrière de l'organe de commutation (22) dans la direction du piston (4), ainsi qu'une pénétration du milieu (53) entre l'organe de commutation (22) et l'élément de retenue (29), également dans le cas d'une sollicitation de pression, à travers le canal d'aiguille, le tourillon de guidage (50) étant toujours guidé dans la voie de guidage (49).

32. Dispositif d'injection selon l'une quelconque des revendications 1 à 31, caractérisé en ce que l'axe longitudinal de l'organe de commutation (22) est situé en position coaxiale à l'axe longitudinal (28) du canal d'aiguille.

33. Dispositif d'injection selon l'une quelconque des revendications 1 à 32, caractérisé en ce que l'axe longitudinal de l'organe de commutation (22) est disposé en position excentrique par rapport à l'axe longitudinal (28) du canal d'aiguille.

34. Dispositif d'injection selon l'une quelconque des revendications 1 à 33, caractérisé en ce que l'organe de comutation (22) dans sa zone superficielle qui s'appuie, dans la position de repos, contre l'élément de retenue (21), respectivement contre l'anneau de support, présente un canal de pénétration (90) s'étendant dans la direction longitudinale de l'organe de commutation (22), dont la section d'écoulement est inférieure à la section de passage minimale pour l'écoulement d'un liquide possédant une viscosité prédéterminable.

35. Dispositif d'injection selon l'une quelconque des revendications 1 à 34, caractérisé en ce que, sur la surface interne (29) de l'anneau de support (26) tournée vers l'organe de commutation (22), est disposé un renfoncement s'étendant approximativement dans la direction longitudinale du cylindre d'injection (2).

36. Dispositif d'injection selon l'une quelconque des revendications 1 à 35, caractérisé en ce que, sur la surface (56) de l'organe de commutation (22) tournée vers l'élément de retenue (21), respectivement sur la surface interne (29) de l'élément de retenue (21) tournée vers l'organe de commutation (22), est disposé au moins un élément faisant saillie, formant une élévation (103), en particulier une nervure s'étendant dans la direction longitudinale du cylindre d'injection (2).
